# EUROPEAN PATENT APPLICATION

(11) **EP 4 245 316 A1**
(43) Date of publication of application: **20.09.2023**
(21) Application number: 21891249.1
(22) Date of filing: 15.11.2021
(51) Int. Cl.: A61K 39/39, A61K 39/25, A61P 31/22, A61P 31/12, A61K 41/10, B82Y 5/00

(54) **ADJUVANT CONTAINING ZINC ALUMINUM RISEDRONATE, AND APPLICATION THEREOF**

(30) Priority: 16.11.2020 CN 202011278492
(71) Applicant: Xiamen University, Xiamen, Fujian 361005 (CN); Xiamen Innovax Biotech Co., Ltd., Xiamen, Fujian 361022 (CN)
(72) Inventor: ZHAO, Qinjian, Xiamen, Fujian 361005 (CN); HUANG, Xiaofen, Xiamen, Fujian 361005 (CN); NIE, Meifeng, Xiamen, Fujian 361005 (CN); ZHANG, Zhigang, Xiamen, Fujian 361005 (CN); YUAN, Quan, Xiamen, Fujian 361005 (CN); ZHANG, Tianying, Xiamen, Fujian 361005 (CN); LI, Shaowei, Xiamen, Fujian 361005 (CN); XIA, Ningshao, Xiamen, Fujian 361005 (CN)
(74) Representative: Meissner Bolte Partnerschaft mbB
(86) International application number: PCT/CN2021/130588
(87) International publication number: WO 2022/100729

(57) **Abstract**

An adjuvant containing zinc aluminum risedronate, an immunogenic composition comprising the adjuvant and an immunogen, and uses of the adjuvant and the immunogenic composition.

## Description

### Technical Field

The present application relates to the technical field of biopharmaceuticals. Specifically, the present application relates to an adjuvant containing zinc aluminum risedronate, an immunogenic composition comprising the adjuvant and an immunogen, and uses of the adjuvant and the immunogenic composition.

### Background Art

Adjuvants are substances or mixtures that can specifically or non-specifically bind to immunogens, and stimulate and induce an organism to produce long-term and effective specific immune responses. The immunobiological effects of adjuvants include reducing dosage of an immunogen, enhancing immunogenicity of antigens, and changing the type of immune response. Adjuvants currently approved for human vaccines include aluminum adjuvant, MF59, AS04, AS01_{B}, CpG1018, etc., and several new adjuvants are in clinical trials, including: AS03, RC-529, Advax, Matrix-M, etc. Aluminum adjuvant is the first adjuvant approved for human vaccines. It has been used for nearly 90 years and is recognized as the most widely used, safe and effective adjuvant. However, with the increase of researched vaccine varieties, it is found that aluminum adjuvant stimulates induction of Th2 immunodominant response, and has a limited effect on inducing Th1 immune response, which also limits the application of aluminum adjuvant in therapeutic vaccines, such as varicella zoster virus vaccine, hepatitis B therapeutic vaccine and tumor vaccine, etc. Moreover, compared with many new vaccine adjuvants, the activity of aluminum adjuvant is weak, and its immune enhancement effect on most genetically engineered antigens other than virus-like particle antigens is not ideal, so that it's application in non-multimerized protein antigen vaccines is restricted.

Aluminum-containing adjuvant system refers to a adjuvant system formed based on aluminum adjuvant and other adjuvant components or presenting systems (e.g., MPL, CPG, QS21 and liposomes, etc.), which can induce relatively balanced Th1 and Th2 immune responses. At present, there are several aluminum-containing adjuvant systems that have been approved for marketing or entered clinical trials (NPJ Vaccines. 2018, 10;3:51.). For example, AS04 is developed by GlaxoSmithKline (GSK), this adjuvant system is based on aluminum adjuvant in which TLR-4 (Toll like receptor 4) agonist: 3-O-deacyl-4'-monophosphoryl lipid A (MPL) is added. Because of its glucosamine phosphorylation, the agonist has a high affinity with Al³⁺, and is adsorbed by aluminum adjuvant to form a complex adjuvant, which can improve the expression of γ-interferon (Interferon-γ, IFN-γ) in antigen-specific CD4⁺ T cells (IFN-γ is one of the important indicators of T cell response). At present, AS04 has been successfully used in hepatitis B vaccine (Fendrix, approved for marketing in 2005) and HPV16/18 bivalent cervical cancer vaccine (Cervarix, approved for marketing in 2007). Compared with using aluminum adjuvant alone, AS04 can significantly increase the neutralizing antibody titer of HPV16/18 bivalent cervical cancer vaccine (Cervarix) and prolong the effective period of immune protection, and when the vaccine is used to immunize once or twice, it could produce similar protective effect as provided by three immunization. CpG, a receptor agonist of TLR-9, has entered clinical trials as an adjuvant for malaria vaccines and hookworm vaccines in combination with aluminum adjuvants.

The adsorption of aluminum adjuvant to antigen is one factor resulting in its immune enhancement effect. Ligand exchange is the strongest interaction between the adjuvant and the antigen, which is produced by the ligand exchange between the phosphate group in the antigen and the hydroxyl group in aluminum hydroxide or aluminum phosphate. This is the concept of "phosphophilicity" of aluminum adjuvant surface proposed by the inventor Zhao in 2001 (Analytical Biochemistry, 2001, 295(1):76-81). The aforementioned AS04, MPL as new type composite adjuvants formed by the adsorption of phosphate groups and aluminum adjuvant have been well used in clinical practice.

Bisphosphonates (BPs) drugs are a class of artificially synthesized pyrophosphate analogues with P-C-P as the central skeleton. As organic bisphosphonates, they have high affinity to ions of calcium, aluminum, zinc, magnesium and so on due to the phosphate groups contained therein, and thus are used for treatment of bone diseases and calcium metabolism diseases, such as osteoporosis, osteitis deformans, and hypercalcemia and bone pain caused by malignant tumor bone metastasis. At the same time, clinical studies have shown that use of bisphosphonate drugs in the adjuvant treatment of multiple myeloma, breast cancer, kidney cancer, prostate cancer, etc. can reduce the incidence of bone-related diseases in patients, the recurrence rate of cancer, and improve the survival period and clinical outcome of patients. In addition, bisphosphonates exhibit adjuvant activity due to its positive immune regulation effect, and patented inventions of using them as immune enhancers in vaccine preparations have also been reported (Chinese patent:CN103768595B; US patent:US20170281759A1; Chinese patent:CN108289902A). Bisphosphonate drugs are usually administered orally or intravenously in clinical practice, which have a strong mucosal stimulating effect, while in the use of vaccines, immunization is usually performed by intramuscular injection. In addition, bisphosphonates have potential side effects and still need further improvement.

Coronavirus (CoV) is an enveloped, non-segmented, single-stranded positive-sense RNA virus, belonging to the subfamily *Orthocoronavirinae,* the family *Coronaviridae,* the order *Nidovirales,* and is divided into α, β, γ and δ four genera. So far, 7 coronaviruses have been found to infect humans, among which the seasonal epidemic coronaviruses include:229E and NL63 of the genus α, OC43 and HKU1 of the genus β, and pandemic coronaviruses: severe acute respiratory syndrome-associated coronavirus (SARS-CoV), Middle East respiratory syndrome-associated coronavirus (MERS-CoV) and novel coronavirus (SARS-CoV-2). It is currently known that coronaviruses can infect vertebrates such as humans, murine, swine, feline, canine, mink, guinea pig, hamster, rhesus monkey, and birds. At the same time, Dutch virologists have confirmed that SARS-CoV-2 can be transmitted from humans to minks and then back to humans, which confirms the animal-to-human transmission chain of SARS-CoV-2. SARS-CoV, MERS-CoV and SARS-CoV-2 have strong transmission ability and pathogenicity. The current global pandemic of SARS-CoV-2 poses a major threat to people's life, health, safety and social activities and causes huge economic losses, and vaccines are the most effective means to control and prevent the infection of the virus.

The coronavirus spike protein (S protein) is a type I transmembrane protein consisting of two parts, the S 1 and S2 protein subunits, which mediates the binding to host cell surface receptors and the fusion of cell membranes to allow the virus to enter target cells. Multiple evidences have shown that antibodies against the spike protein may play a key role in the immune prevention and treatment of pneumonia caused by SARS-CoV-2, and thus the spike protein is currently the key target for the development and design of recombinant protein vaccines against SARS-CoV-2. Studies have found that acute SARS-CoV-2 infection can weaken the long-lasting neutralizing antibody response, but still achieve immune memory through virus-specific memory T cells (Cell, 2020, 183(1):13-15), indicating the importance of T cell responses in the prevention of SARS-CoV-2 infection. The subunit protein vaccine prepared by recombinant DNA technology has good safety and can achieve multi-shot booster immunization, but its immunogenicity is weak. At the same time, the World Health Organization (WHO) formulated the target product characteristics of the 2019 coronavirus disease (COVID-19) vaccine in April 2020. In the event of an outbreak, the vaccine must take effect quickly, provide protection within 2 weeks, achieve basic immunity with 1 dose, not exceed 2 doses, and provide at least 6 months of protection in terms of durability.

Therefore, there is still a need in the art to develop new adjuvants to improve the efficacy of vaccines (e.g., COVID-19 vaccines).

### Contents of the present invention

After in-depth research, the inventors of the present application have developed a new type of adjuvant containing zinc aluminum risedronate, which can be used as a vaccine adjuvant or drug delivery carrier, etc., and can be used to effectively improve immunogenicity of antigens.

In particular, it has been surprisingly found that when the adjuvant of the present application is used in combination with an immunogen (e.g., SARS-CoV-2 S protein), it can effectively stimulate or induce high levels of functional antibodies and balanced cellular and humoral immune responses in various animals such as Balb/c mice, mink, guinea pig, Syrian golden hamster and cynomolgus monkey. Therefore, the adjuvant of the present application can improve the druggability of vaccines (e.g., SARS-CoV-2 S protein vaccines).

In addition, it has also been found that when the adjuvant of the present application is used in combination with SARS-CoV-2 S protein, it can effectively prevent the infection of SARS-CoV-2. For example, the vaccine containing the adjuvant of the present application and SARS-CoV-2 S protein can effectively prevent or reduce the replication of SARS-CoV-2 in the upper and lower respiratory tracts of animals (e.g., mink), and prevent SARS-CoV-2 from infecting and damaging the lungs of animals (e.g., mink).

### Adjuvant

In one aspect, the present application provides an adjuvant comprising zinc aluminum risedronate (also referred to herein as zinc aluminum risedronate adjuvant), which has a zinc:risedronate molar concentration ratio in the range of 1:1 to 16:1 (e.g., 2:1 to 16:1); and, a zinc:aluminum molar ratio in the range of 1:1 to 50:1 (e.g., 5:1 to 50:1).

In certain embodiments, zinc aluminum risedronate is present in particulate form. In certain embodiments, zinc aluminum risedronate is present in the form of nanoparticles or microparticles. In some embodiments, the particles have a particle size of 0.01-100 µm, such as 0.01-60 µm, 0.01-50 µm, 0.1-60 µm, 0.1-30 µm, 0.4-30 µm, 0.4-20 µm.

In some embodiments, the adjuvant has a zinc:risedronate molar concentration ratio in the range of 1:1 to 2:1, 2:1 to 4:1, 4:1 to 6:1, 6:1 to 8:1, 8:1 to 10:1, 10:1 to 12:1, 12:1 to 14:1 or 14:1 to 16:1. In certain embodiments, the adjuvant has a zinc:risedronate molar concentration ratio of at least 1:1, at least 2:1, at least 3:1, at least 4:1, at least 5:1, at least 6:1, at least 7:1, at least 8:1, at least 10:1, at least 12:1, at least 14:1, or at least 15:1. In certain embodiments, the adjuvant has a zinc:risedronate molar concentration ratio of no more than 16:1, no more than 14:1, no more than 12:1, no more than 10:1, no more than 8:1, no more than 7:1, no more than 6:1, no more than 5:1, no more than 4:1, no more than 3:1, or no more than 2:1.

In certain embodiments, the adjuvant has a zinc:risedronate molar concentration ratio of 1:1, 2:1, 4:1, 4.5:1, 6:1, 8:1, 10:1, 12:1, 14:1 or 16:1. In certain embodiments, the adjuvant has a zinc:risedronate molar concentration ratio of 4:1 or 4.5:1.

In certain embodiments, the adjuvant has a zinc:aluminum molar concentration ratio in the range of 1:1 to 2:1, 2:1 to 3:1, 3:1 to 4:1, 4:1 to 5:1, 5:1 to 6:1, 6:1 to 8:1, 8:1 to 10:1, 10:1 to 12:1, 12:1 to 15:1, 15:1 to 20:1, 20:1 to 30:1, 30:1 to 40:1, or 40:1 to 50:1. In certain embodiments, the adjuvant has a zinc:aluminum molar concentration ratio of at least 1:1, at least 2:1, at least 3:1, at least 4:1, at least 5:1, at least 6:1, at least 8:1, at least 10:1, at least 12:1, at least 15:1, at least 20:1, at least 30:1, or at least 40:1. In certain embodiments, the adjuvant has a zinc:aluminum molar concentration ratio of no more than 50:1, no more than 40:1, no more than 30:1, no more than 20:1, no more than 15:1, no more than 12:1, no more than 10:1, no more than 8:1, no more than 6:1, no more than 5:1, no more than 4:1, no more than 3:1, or no more than 2:1.

In some embodiments, the adjuvant has a zinc:aluminum molar concentration ratio of 50:1, 40:1, 30:1, 20:1, 15:1, 12:1, 10:1, 8:1, 6:1, 5:1, 4:1, 3:1, 2:1, or 1:1. In certain embodiments, the adjuvant has a zinc:aluminum molar concentration ratio of 10:1.

In certain embodiments, the adjuvant has a zinc:risedronate molar ratio in the range of 2:1 to 8:1, such as 2:1 to 4:1, 4:1 to 6:1, or 6:1 to 8:1, and the zinc:aluminum molar concentration ratio is in the range of 2:1 to 50:1 (e.g. in the range of 5:1 to 20:1), for example 2:1 to 3:1, 3:1 to 4:1, 4:1 to 5:1, 5:1 to 6:1, 6:1 to 8:1, 8:1 to 10:1, 10:1 to 12:1, 12:1 to 15:1, or 15:1 to 20:1.

In certain embodiments, the adjuvant has a zinc:risedronate molar ratio of 4:1 and a zinc:aluminum molar ratio of 10:1.

In certain embodiments, the adjuvant has a zinc:risedronate molar ratio of 4.5:1 and a zinc:aluminum molar ratio of 10:1.

In certain embodiments, the adjuvant has a pH of 5.0-8.0, for example, 5.0-7.0, 5.0-5.5, 5.5-6.0, 6.0-6.5, 6.5-7.0, 7.0-7.5, or 7.5-8.0. In certain embodiments, the adjuvant has a pH of 7.0-7.5. In certain embodiments, the adjuvant has a pH of 5.5-6.5. In certain embodiments, the adjuvant has a pH of 5.8-6.3.

In certain embodiments, the adjuvant has a zero charge point of 3.0-8.0, such as 4.0-8.0, 3.0-4.0, 4.0-5.0, 5.0-6.0, 6.0-7.0, or 7.0-8.0. In some embodiments, the adjuvant has a zero charge point of 4.0-6.0.

The adjuvant of the present application has a good adsorption rate for an immunogen (e.g., a protein). In certain embodiments, the adjuvant of the present invention has an adsorption rate of at least 60%, at least 70%, at least 75%, at least 80%, at least 85%, at least 90%, or at least 95% for an immunogen (e.g., a protein).

### Immunogenic composition

Without being limited by theory, the adjuvant of the present invention can be used in combination with various immunogens to enhance the immunogenicity of the immunogens. Such immunogens include, but are not limited to, proteins, nucleic acids, polysaccharides, or immunogenic portions thereof, and the like.

Accordingly, in one aspect, the present application provides an immunogenic composition comprising an immunogen and the adjuvant as described above.

In some embodiments, the immunogen includes but is not limited to a protein, nucleic acid, polysaccharide, or immunogenic part thereof, etc. In certain embodiments, the immunogen is derived from a pathogen such as a virus, bacterium, and fungus.

In certain embodiments, the immunogen is a protein or immunogenic fragment thereof, such as a protein or immunogenic fragment thereof derived from a pathogen such as a virus, bacterium, or fungus. In certain embodiments, the virus includes, but is not limited to, respiratory virus (e.g., influenza, parainfluenza, rhinovirus, coronavirus, respiratory syncytial virus), enterovirus (e.g., EV71 virus, rotavirus), varicella zoster virus (VZV).

In certain embodiments, the immunogen is a coronavirus protein (e.g., spike protein) or immunogenic fragment thereof. In some embodiments, the coronavirus is selected from the group consisting of Orthocoronavirinae α virus (e.g., 229E and NL63), Orthocoronavirinae β virus (e.g., OC43 and HKU1), severe acute respiratory syndrome-associated coronavirus (SARS-CoV), Middle East respiratory syndrome-related coronavirus (MERS-CoV), and SARS-CoV-2. In certain embodiments, the coronavirus is selected from the group consisting of severe acute respiratory syndrome-associated coronavirus (SARS-CoV), Middle East respiratory syndrome-associated coronavirus (MERS-CoV) and SARS-CoV-2. In certain embodiments, the coronavirus is SARS-CoV-2. In some embodiments, the immunogen is a structural protein of SARS-CoV-2 or an immunogenic fragment thereof, such as S protein or an immunogenic fragment thereof.

In certain embodiments, the immunogen is a varicella zoster virus protein or an immunogenic fragment thereof, such as gE protein of varicella zoster virus or an immunogenic fragment thereof.

In certain embodiments, the immunogen is an influenza virus protein or an immunogenic fragment thereof, such as HA protein of influenza virus or an immunogenic fragment thereof.

In certain embodiments, the immunogen is a rotavirus protein or an immunogenic fragment thereof, such as VP4 protein of rotavirus or an immunogenic fragment thereof.

In certain embodiments, the immunogenic composition further comprises a pharmaceutically acceptable auxiliary material, such as excipient, preservative, antibacterial agent, buffer and/or additional immune adjuvant. In certain embodiments, the additional immune adjuvant is selected from the group consisting of aluminum adjuvant (e.g., aluminum hydroxide), Freund's adjuvant (e.g., complete Freund's adjuvant and incomplete Freund's adjuvant), *Corynebacterium pumilus,* lipopolysaccharide, cytokine, or any combination thereof.

In certain embodiments, the immunogenic composition further comprises a second immunogen. In certain embodiments, the second immunogen includes, but is not limited to, a protein, nucleic acid, polysaccharide, or immunogenic portion thereof, and the like.

In certain embodiments, the immunogenic composition is a vaccine.

### Method for preparing adjuvant

In one aspect, the present application provides a method for preparing the adjuvant as described above, comprising the steps of:
1) providing a soluble salt solution containing zinc ion and aluminum ion;
2) mixing the soluble salt solution in step (1) with an alkaline risedronate solution to obtain the adjuvant.

In some embodiments, the method further comprises a step of sterilizing the adjuvant obtained in step (2). In certain embodiments, the adjuvant is sterilized by a filter sterilization or a high-temperature and high-pressure sterilization. In certain embodiments, the adjuvant is sterilized by sterilization at 121°C for at least 15 minutes (e.g., at least 30 minutes, for example, 30-60 minutes).

In certain embodiments, the soluble salt solution has a zinc:aluminum molar concentration ratio in the range of 1:1 to 50:1 (e.g., in the range of 5:1 to 50:1). In certain embodiments, the zinc:aluminum molar concentration ratio is in the range of 1:1 to 2:1, 2:1 to 3:1, 3:1 to 4:1, 4:1 to 5:1, 5:1 to 6:1, 6:1 to 8:1, 8:1 to 10:1, 10:1 to 12:1, 12:1 to 15:1, 15:1 to 20:1, 20:1 to 30:1, 30:1 to 40:1, or 40:1 to 50:1. In certain embodiments, the zinc:aluminum molar concentration ratio is at least 1:1, at least 2:1, at least 3:1, at least 4:1, at least 5:1, at least 6:1, at least 8:1, at least 10:1, at least 12:1, at least 15:1, at least 20:1, at least 30:1, or at least 40:1. In certain embodiments, the zinc:aluminum molar concentration ratio is no more than 50:1, no more than 40:1, no more than 30:1, no more than 20:1, no more than 15:1, no more than 12:1, no more than 10:1, no more than 8:1, no more than 6:1, no more than 5:1, no more than 4:1, no more than 3:1, or no more than 2:1.

In certain embodiments, the soluble salt solution has a zinc:aluminum molar concentration ratio of 50:1, 40:1, 30:1, 20:1, 15:1, 12:1, 10:1, 8:1, 6:1, 5:1, 4:1, 3:1, 2:1, or 1:1. In certain embodiments, the soluble salt solution has a zinc:aluminum molar concentration ratio of 10:1.

In certain embodiments, in step (2), the soluble salt solution is mixed with alkaline risedronate solution at a zinc:risedronate molar concentration ratio in the range of 1:1 to 16:1 (e.g., in the range of 2:1 to 16:1),. In some embodiments, the zinc:risedronate molar concentration ratio is in the range of 1:1 to 2:1, 2:1 to 4:1, 4:1 to 6:1, 6:1 to 8:1, 8:1 to 10:1, 10:1 to 12:1, 12:1 to 14:1 or 14:1 to 16:1. In certain embodiments, the zinc:risedronate molar concentration ratio is at least 1:1, at least 2:1, at least 3:1, at least 4:1, at least 5:1, at least 6:1, at least 7:1, at least 8:1, at least 10:1, at least 12:1, at least 14:1, or at least 15:1. In certain embodiments, the zinc:risedronate molar concentration ratio is no more than 16:1, no more than 14:1, no more than 12:1, no more than 10:1, no more than 8:1, no more than 7:1, no more than 6:1, no more than 5:1, no more than 4:1, no more than 3:1, or no more than 2:1.

In certain embodiments, the zinc:risedronate molar concentration ratio is 1:1, 2:1, 4:1, 4.5:1, 6:1, 8:1, 10:1, 12:1, 14:1 or 16:1. In certain embodiments, the zinc:risedronate molar concentration ratio is 4:1 or 4.5:1.

In certain embodiments, in step (2), the soluble salt solution is mixed with the alkaline risedronate solution in a manner allowing the co-precipitation of zinc ion, aluminum ion and risedronate. In some embodiments, in step (2), the alkaline risedronate solution is added dropwise to the soluble salt solution to allow the co-precipitation of zinc ion, aluminum ion and risedronate. In certain embodiments, in step (2), during the process of mixing the soluble salt solution with the alkaline risedronate solution, the co-precipitation of zinc ion, aluminum ion and risedronate occurs to produce zinc aluminum risedronate particles. In certain embodiments, the produced zinc aluminum risedronate particles have a particle size of 0.01-100 µm, such as 0.01-60 µm, 0.01-50 µm, 0.1-60 µm, 0.1-30 µm, 0.4-30 µm, 0.4- 20 µm.

In some embodiments, the alkaline risedronate solution is selected from the group consisting of solution of risedronic acid and sodium hydroxide, solution of risedronic acid and phosphate (e.g., solution of risedronic acid and disodium hydrogen phosphate, solution of risedronic acid and sodium dihydrogen phosphate), or any combination thereof. In some embodiments, the alkaline risedronate solution is solution of risedronic acid and sodium hydroxide or solution of risedronic acid and disodium hydrogen phosphate.

In some embodiments, the soluble salt solution in step (1) is selected from, for example, sulfate solution, chlorate solution, acetate solution, or any combination thereof. In certain embodiments, the soluble salt solution is a chlorate solution or an acetate solution.

### Use of adjuvant

In one aspect, the present application further provides a use of the adjuvant in the manufacture of an immunogenic composition, or as a carrier for delivering an immunogen, or as an immunoenhancer for an immunogen. In one aspect, the present application also provides a use of the adjuvant for enhancing immunogenicity of an immunogen. In one aspect, the present application also provides a use of the adjuvant for enhancing an immune response of a subject to an immunogen. In one aspect, the present application also provides a use of the adjuvant in the manufacture of a preparation for enhancing an immune response of a subject to an immunogen. It is easy to understand that the various descriptions above for the adjuvant and immunogen and so on can also be applied to these aspects as well.

In certain embodiments, the immune response is a cellular immune response and/or a humoral immune response. In certain embodiments, the cellular immune response is a T cell immune response. In certain embodiments, the T cell immune response is a Th1 and/or Th2 immune response.

### Method for preparing immunogenic composition/method for enhancing immunogenicity of immunogen

In one aspect, the present application also provides a method for preparing an immunogenic composition, which comprises: a step of mixing the adjuvant with an immunogen. In one aspect, the present application provides a method for enhancing immunogenicity of an immunogen, which comprises a step of mixing the immunogen with the adjuvant. It is easy to understand that the adjuvant of the present invention can be used in combination with various possible immunogens to enhance the immunogenicity of the immunogens. Accordingly, the various descriptions above for the adjuvants, immunogens and so on can also be applied to these aspects as well.

In certain embodiments, the immunogen includes, but is not limited to, a protein, nucleic acid, polysaccharide, or immunogenic portion thereof, and the like. In certain embodiments, the immunogen is derived from a pathogen such as virus, bacterium, and fungus.

In certain embodiments, the immunogen is a protein or immunogenic fragment thereof, such as a protein or immunogenic fragment thereof derived from a pathogen such as virus, bacterium, or fungus. In certain embodiments, the virus includes, but is not limited to, respiratory virus (e.g., influenza, parainfluenza, rhinovirus, coronavirus, respiratory syncytial virus), enterovirus (e.g., EV71 virus, rotavirus), varicella zoster virus (VZV).

In certain embodiments, the immunogen is a coronavirus protein (e.g., spike protein) or an immunogenic fragment thereof. In some embodiments, the coronavirus is selected from the group consisting of Orthocoronavirinae α virus (e.g., 229E and NL63), Orthocoronavirinae β virus (e.g., OC43 and HKU1), severe acute respiratory syndrome-associated coronavirus (SARS-CoV), Middle East respiratory syndrome-related coronavirus (MERS-CoV), and SARS-CoV-2. In certain embodiments, the coronavirus is selected from the group consisting of severe acute respiratory syndrome-associated coronavirus (SARS-CoV), Middle East respiratory syndrome-associated coronavirus (MERS-CoV) and SARS-CoV-2. In certain embodiments, the coronavirus is SARS-CoV-2. In some embodiments, the immunogen is a structural protein of SARS-CoV-2 or an immunogenic fragment thereof, such as S protein or an immunogenic fragment thereof.

In certain embodiments, the immunogen is a protein of varicella zoster virus or an immunogenic fragment thereof, such as gE protein of varicella zoster virus or an immunogenic fragment thereof.

In certain embodiments, the immunogen is a protein of influenza virus or an immunogenic fragment thereof, such as HA protein of influenza virus or an immunogenic fragment thereof.

In certain embodiments, the immunogen is a protein of rotavirus or an immunogenic fragment thereof, such as VP4 protein of rotavirus or an immunogenic fragment thereof.

In some embodiments, the method further comprises a step of adding a pharmaceutically acceptable auxiliary material. In certain embodiments, the auxiliary material is selected from, for example, excipient, preservative, antibacterial agent, buffer and/or additional immune adjuvant. In certain embodiments, the additional immune adjuvant is selected from the group consisting of aluminum adjuvant (e.g., aluminum hydroxide), Freund's adjuvant (e.g., complete Freund's adjuvant and incomplete Freund's adjuvant), *Corynebacterium pumilus,* lipopolysaccharide, cytokine, or any combination thereof.

In certain embodiments, the method further comprises a step of adding a second immunogen. In certain embodiments, the second immunogen includes, but is not limited to, a protein, nucleic acid, polysaccharide, or immunogenic portion thereof, and the like.

In certain embodiments, the immunogenic composition is a vaccine.

### Use of immunogenic composition

In one aspect, the present application provides a use of the immunogenic composition in the manufacture of a medicament for preventing and/or treating a disease in a subject, the disease being a disease to be prevented or treated by an immune response induced by the immunogen.

It is easy to understand that the various descriptions above for the adjuvant, immunogen, immunogenic composition and so on are also applicable to this aspect. It is also readily understood that various immunogens can be included in the immunogenic composition; accordingly, the prepared medicament can be used to prevent or treat various corresponding diseases. For example, when the immunogenic composition comprises an immunogen derived from a pathogen (e.g., a virus), the immunogenic composition of the present application can be used to prepare a medicament for preventing and/or treating the pathogen infection (e.g., viral infection) or a disease associated with the pathogen infection (e.g., viral infection).

In certain embodiments, the immunogen is derived from a coronavirus (e.g., SARS-CoV-2), and the disease is a coronavirus (e.g., SARS-CoV-2) infection or a coronavirus (e.g., SARS-CoV-2) infection-associated disease. In certain embodiments, the immunogen is a coronavirus (e.g., SARS-CoV-2) protein (e.g., spike protein) or an immunogenic fragment thereof, and the disease is a coronavirus (e.g., SARS-CoV-2) infection or a coronavirus (e.g., SARS-CoV-2) infection-associated disease.

In some embodiments, the immunogen is a SARS-CoV-2 structural protein (e.g., S protein) or an immunogenic fragment thereof; and, the disease is a SARS-CoV-2 infection or a SARS-CoV-2 infection-associated disease (e.g., pneumonia caused by SARS-CoV-2 (COVID-19)).

In certain embodiments, the immunogen is derived from varicella zoster virus, and the disease is a varicella zoster virus infection or a varicella zoster virus infection-associated disease.

In certain embodiments, the immunogen is a varicella zoster virus protein (e.g., gE protein) or an immunogenic fragment thereof, and the disease is a varicella zoster virus infection-associated disease.

In certain embodiments, the immunogen is derived from influenza virus, and the disease is an influenza virus infection or an influenza virus infection-associated disease.

In certain embodiments, the immunogen is an influenza virus protein (e.g., HA protein) or an immunogenic fragment thereof, and the disease is an influenza virus infection or an influenza virus infection-associated disease.

In certain embodiments, the immunogen is derived from rotavirus, and the disease is a rotavirus infection or a rotavirus infection-associated disease.

In certain embodiments, the immunogen is a rotavirus protein (e.g., VP4 protein) or an immunogenic fragment thereof, and the disease is a rotavirus infection or a rotavirus infection-associated disease.

In certain embodiments, the subject is an animal, such as an avian or mammal. In certain embodiments, the subject is a rodent, porcine, feline, canine, equine, primate, or avian. In certain embodiments, the subject is a non-human subject. In certain embodiments, the subject is a mouse, mink, guinea pig, Syrian golden hamster, or cynomolgus monkey. In certain embodiments, the subject is a human.

### Method for stimulating/enhancing immune response

In one aspect, the present application provides a method for stimulating or enhancing an immune response to an immunogen in a subject, which comprises a step of administering to the subject an effective amount of an immunogenic composition comprising the immunogen and the adjuvant of the present invention. It is easy to understand that various descriptions above of the adjuvant, immunogen, immunogenic composition, subject and so on are also applicable to this aspect.

In certain embodiments, the immune response is a cellular immune response and/or a humoral immune response. In certain embodiments, the cellular immune response is a T cell immune response. In certain embodiments, the T cell immune response is a Th1 and/or Th2 immune response.

In certain embodiments, the immunogenic composition is administered by a route selected from the group consisting of intramuscular injection, subcutaneous injection, intradermal administration, intranasal administration, oral administration, transdermal administration, or intravenous injection. In certain embodiments, the immunogenic composition is administered by intramuscular injection.

### Method for disease treatment/prevention

In one aspect, the present application provides a method for preventing or treating a disease, comprising a step of administering to a subject an effective amount of an immunogenic composition, wherein the disease is a disease to be prevented or treated by an immune response induced by the immunogen.

It is easy to understand that various descriptions above of the adjuvant, immunogen, immunogenic composition, subject and so on are also applicable to this aspect. It is also readily understood that various immunogens can be contained in the immunogenic composition; accordingly, the immunogenic composition can be used to prevent or treat various corresponding diseases. For example, when the immunogenic composition contains an immunogen derived from a pathogen (e.g., a virus), the immunogenic composition of the present application can be used for preventing and/or treating a pathogenic infection (e.g., a viral infection) or a pathogenic infection (e.g, a viral infection)-associated disease in a subject.

In some embodiments, the disease is a coronavirus (e.g., SARS-CoV-2) infection or a coronavirus (e.g., SARS-CoV-2) infection-associated disease. Accordingly, in such embodiments, the immunogen is derived from a coronavirus (e.g., SARS-CoV-2). In certain embodiments, the immunogen is a coronavirus (e.g., SARS-CoV-2) protein (e.g., spike protein) or an immunogenic fragment thereof.

In some embodiments, the disease is a SARS-CoV-2 infection or a SARS-CoV-2 infection-associated disease, such as pneumonia caused by SARS-CoV-2 (COVID-19). Accordingly, in such embodiments, the immunogen is derived from SARS-CoV-2. In certain embodiments, the immunogen is a SARS-CoV-2 structural protein (e.g., S protein) or an immunogenic fragment thereof.

In certain embodiments, the disease is a varicella-zoster virus infection or a varicella-zoster virus infection-associated disease. Accordingly, in such embodiments, the immunogen is derived from varicella zoster virus. In certain embodiments, the immunogen is a varicella-zoster virus protein (e.g., gE protein) or an immunogenic fragment thereof.

In certain embodiments, the disease is an influenza virus infection or an influenza virus infection-associated disease. Accordingly, in such embodiments, the immunogen is derived from influenza virus. In certain embodiments, the immunogen is a influenza virus protein (e.g., HA protein) or an immunogenic fragment thereof.

In certain embodiments, the disease is a rotavirus infection or a rotavirus infection-associated disease. Accordingly, in such embodiments, the immunogen is derived from rotavirus. In certain embodiments, the immunogen is a rotavirus protein (e.g., VP4 protein) or an immunogenic fragment thereof.

In certain embodiments, the subject is an animal, such as an avian or mammal. In certain embodiments, the subject is a rodent, porcine, feline, canine, equine, primate, or avian. In certain embodiments, the subject is a non-human subject. In certain embodiments, the subject is a mouse, mink, guinea pig, Syrian golden hamster, or cynomolgus monkey. In certain embodiments, the subject is a human.

### Description and explanation of relevant terms in the present application

In the present application, unless otherwise stated, scientific and technical terms used herein have the meanings commonly understood by those skilled in the art.

According to the present invention, the term "S protein" refers to SARS-CoV-2 spike protein, which belongs to type I transmembrane protein and consists of two parts, S1 and S2 protein subunits, and which mediates the binding to host cell surface receptors and the fusion with cell membranes so as to allow the virus to enter target cells. The amino acid sequence of the wild-type S protein is known to those skilled in the art, and its typical amino acid sequence can be found in Genbank:QHD43416.1. In some embodiments, the amino acid sequence of the wild-type S protein is shown in SEQ ID NO:1.

It is easy to understand that those skilled in the art can modify the wild-type S protein, for example, by removing the furin cleavage site, increasing the protein multimerization domain (e.g., increasing the trimerization domain of the T4 bacteriophage fibritin protein), and/or, adding a tag to get a desired performance. In certain embodiments, the amino acid sequence of the modified S protein is shown in SEQ ID NO:4.

Unless otherwise indicated herein or clearly contradicted by the context, the term "S protein" shall be construed to cover the wild-type S protein as well as modified S protein.

According to the present invention, "gE protein" is an envelope glycoprotein of varicella-zoster virus (VZV). The amino acid sequence of the wild-type gE protein is known to those skilled in the art, and its typical amino acid sequence can be found in Genbank:DQ008355.1. In some embodiments, the amino acid sequence of the gE protein is shown in SEQ ID NO:2.

According to the present invention, the term "HA protein" refers to an influenza virus hemagglutinin (HA), which is a key protein that mediates virus invasion into host cells and is also the main target of anti-influenza virus neutralizing antibodies. The amino acid sequence of the HA protein of each subtype of influenza virus is well known to those skilled in the art. For example, a typical amino acid sequence of the type B Victoria-lineage influenza virus can be found in Genbank:AIU46088. In certain embodiments, the amino acid sequence of the HA protein is shown in SEQ ID NO:3.

According to the present invention, "VP4 protein" is an outer layer capsid protein of rotavirus. VP4 protein is an important neutralizing antigen that can stimulate an organismto produce neutralizing antibodies. The amino acid sequence of the wild-type VP4 protein is known to those skilled in the art, and its typical amino acid sequence can be found in Genbank:KP752474 or GenBank:MG729832.

Unless otherwise indicated herein or clearly contradicted by context, the gE protein, HA protein, and VP4 protein described herein should be construed to cover their corresponding wild-type proteins as well as their corresponding modified proteins.

According to the present invention, the term "adjuvant" refers to a non-specific immune enhancer, which can enhance an immune response of a subject to an antigen or change the type of immune response when it is delivered into the subject together with the antigen or in advance. There are many kinds of adjuvants, including but not limited to, aluminum adjuvant (e.g., aluminum hydroxide), Freund's adjuvant (e.g., complete Freund's adjuvant and incomplete Freund's adjuvant), *Corynebacterium pumilus,* lipopolysaccharide, cytokines, etc. Freund's adjuvant is the most commonly used adjuvant in animal experiments. Aluminum hydroxide adjuvant is widely used in clinical trials.

According to the present invention, the term "pharmaceutically acceptable auxiliary material " can be, for example, inert diluent, such as water or other solvent, solubilizer and emulsifier, such as ethanol, isopropanol, ethyl carbonate, ethyl acetate, benzyl alcohol, benzyl benzoate, propylene glycol, 1,3-butanediol, oil (specifically cottonseed oil, peanut oil, corn oil, germ oil, olive oil, castor oil, and sesame oil), glycerin, tetrahydrofurfuryl alcohol, polyethylene glycol, fatty acid ester of sorbitan, and mixture thereof; besides inert diluent, the oral composition can also contain an auxiliary material such as wetting agent, emulsifying and suspending agent, sweetening agent, flavoring agent, coloring agent, fragrance agent and preservative; suspension formulation may also contain suspending agent such as ethoxylated isostearyl alcohol, polyoxyethylene sorbitol and sorbitan ester, microcrystalline cellulose, aluminum metahydroxide, bentonite, agar and tragacanth and mixture thereof; these compositions may also contain auxiliary material such as wetting agent, emulsifying agent and dispersing agent. It may also be desirable to include isotonic agent, such as sugar, sodium chloride, and the like into the compositions. In addition, prolonged absorption of an injectable pharmaceutical form can be brought about by incorporation of a substance, for example, aluminum monostearate and gelatin, which can delay absorption.

As used herein, the term "immunogenicity" refers to an ability to stimulate a subject to form a specific antibody or sensitize lymphocyte. It not only refers to the characteristics of an antigen to stimulate specific immune cells, to activate, proliferate and differentiate immune cells, and to finally produce substances with an immune effect such as antibodies and sensitized lymphocytes, but also refers to the ability of an antigen to stimulate a subject and induce the specific immune response in the subject by which the immune system of the subject produces antibodies or sensitized T lymphocytes. Immunogenicity is the most important property of an antigen. Whether an antigen can successfully induce an immune response in a host depends on three factors: nature of the antigen, reactivity of the host, and immunization method.

According to the present invention, the term "immunogenic fragment" refers to a polypeptide fragment which at least partially retains the immunogenicity of the protein from which it was derived. For example, an immunogenic fragment of the coronavirus spike protein (S protein) refers to a fragment of the coronavirus spike protein that at least partially retains the immunogenicity of the S protein.

According to the present invention, the term "additional immune adjuvant" includes but is not limited to stabilizer; emulsifier; pH regulator, such as sodium hydroxide, hydrochloric acid, etc.; liposome; iscom adjuvant; synthetic glycopeptide, such as muramyl dipeptide; bulking agent such as dextran; carbopol; bacterial cell wall such as mycobacterial cell wall extract; their derivatives such as *Corynebacterium parvum; Propionibacterium acne; Mycobacterium bovis,* such as *Bovine Calmette Guerin* (BCG); vaccinia or animal pox virus protein; subviral particle adjuvant, such as orbivirus; cholera toxin; N,N-dioctadecyl-N',N'-di-(2-hydroxyethyl)-propylene diamine (pyridine); monophosphoryl lipid A; dimethyldioctadecylammonium bromide; their compositions and mixtures. Examples of suitable stabilizer include, but are not limited to, sucrose, gelatin, peptone, digested protein extract such as NZ-amine or NZ-amine AS. Examples of emulsifier include, but are not limited to, mineral oil, vegetable oil, peanut oil, and other standard, metabolizable, nontoxic oils useful in injectable or intranasal vaccine compositions.

As used herein, the term "subject" may be any animal, such as an avian or mammal. In certain embodiments, the subject is a rodent, porcine, feline, canine, equine, primate, or avian. In certain embodiments, the subject is a non-human subject. In certain embodiments, the subject is a mouse, mink, guinea pig, Syrian golden hamster, or cynomolgus monkey. In certain embodiments, the subject is a human.

As used herein, the terms "pneumonia caused by SARS-CoV-2" and "COVID-19" refer to a pneumonia caused by SARS-CoV-2 infection, and both have the same meaning.

According to the present invention, the term "effective amount" refers to an amount sufficient to obtain, or at least partly obtain, the desired effect. For example, an effective amount for preventing a disease (e.g., COVID-19) refers to an amount sufficient to prevent, arrest, or delay the occurrence of a disease (e.g., COVID-19); an effective amount for treating a disease refers to an amount sufficient to cure or at least partially prevent the occurrence of the disease and its complications in a patient with the disease. Determining such an effective amount is well within the capability of those skilled in the art. For example, an amount effective for therapeutic use will depend on the severity of the disease to be treated, the general state of the patient's own immune system, the general condition of the patient such as age, weight and sex, the mode of administration of the drug, and other therapies administered concomitantly etc.

### Beneficial effects of the present invention

Compared with the prior art, the present invention provides an adjuvant containing zinc aluminum risedronate, which can be used as a vaccine adjuvant or drug delivery carrier, etc., and can be used to effectively improve immunogenicity of antigens, and its effect is better than that of the Aluminum adjuvant.

In particular, when the adjuvant of the present application is used in combination with an immunogen (e.g., SARS-CoV-2 S protein), it can stimulate or induce high levels of functional antibodies and balanced cellular and humoral immune responses in various animals, such as Balb/c mice, mink, guinea pig, Syrian golden hamster, and cynomolgus monkey. Therefore, the adjuvant of the present application can improve the druggability of vaccines (e.g., SARS-CoV-2 S protein vaccines).

### Brief Description of the Drawings

Fig. 1 shows the pictures of aluminum adjuvant (A1001) (Fig. 1A) and zinc aluminum risedronate adjuvant (FH002C) (Fig. 1B) in terms of suspension appearances and particle morphology under optical microscope and electron microscope.
Fig. 2 shows the measurement results of particle sizes of Al001 (Fig. 2A) and FH002C (Fig. 2B); n=3, mean±SD.
Fig. 3 shows the measurement results of Zeta potentials of Al001 and FH002C at different pH values; n=3, mean±SEM.
Fig. 4A to Fig. 4F show the pictures of FH002C adjuvants with different ratios of zinc, aluminum and risedronate in terms of suspension appearance and particle morphology under electron microscope.
Fig. 5 shows the antigenicity detection results of the recombinant SARS-CoV-2 S protein without adjuvant and the dissociated vaccine preparation (FH002C combined with recombinant S protein), wherein the ELISA detection results based on the specific binding of ACE2 receptor at different protein concentrations are shown.
Fig. 6 shows the detection results of antibody binding titer and neutralization titer in the mouse sera from the mice immunized with different immunogen components (1 µg of recombinant S protein, 10 µg of recombinant S protein, 1 µg of recombinant S protein + Al001, 10 µg of recombinant S protein + Al001, 1 µg of recombinant S protein + FH002C, and 10 µg of recombinant S protein + FH002C) respectively; *p<0.05; **p<0.01; ***p<0.001; ****p<0.0001; n=5, mean±SEM.
Fig. 7 shows the specific antibody binding titer results of the Balb/C mice immunized with FH002C adjuvants with different ratios of zinc, aluminum, and risedronate combined with recombinant SARS-CoV-2 S protein; * p<0.05; ** p<0.01; ***p<0.001; n=5, mean±SEM. Note: the abscissa represents the molar concentration ratio of zinc:aluminum:risedronate.
Fig. 8 shows the specific antibody binding titer results of the Syrian golden hamsters immunized with FH002C or Al001 combined with recombinant SARS-CoV-2 S protein, *p<0.05; **p<0.01; ***p<0.001; ^{∗∗∗∗}p<0.0001; n=22 or 16, Geometric mean ± Geometric mean SD.
Fig. 9 shows the specific antibody binding titer results of the cynomolgus monkeys immunized with FH002C or Al001 combined with recombinant SARS-CoV-2 S protein, n=2, Geometric mean±Geometric mean SD.
Fig. 10 shows the serum neutralizing antibody titer results of the Syrian golden hamsters immunized with FH002C or Al001 combined with recombinant SARS-CoV-2 S protein, *p<0.05; **p<0.01; ***p<0.001; ****p <0.0001; n=22 or 16, Geometric mean ± Geometric mean SD.
Fig. 11 shows the neutralizing antibody titer results of the cynomolgus monkeys immunized with FH002C or Al001 combined with recombinant SARS-CoV-2 S protein, n=2, Geometric mean±Geometric mean SD.
Fig. 12 shows the serum blocking titer results of the Syrian golden hamsters immunized with FH002C or Al001 combined with recombinant SARS-CoV-2 S protein, *p<0.05; **p<0.01; ***p<0.001; ****p<0.0001; n=22 or 16, Geometric mean ± Geometric mean SD.
Fig. 13 shows the serum blocking titer results of the cynomolgus monkeys immunized with FH002C or Al001 combined with recombinant SARS-CoV-2 S protein, n=2, Geometric mean±Geometric mean SD.
Fig. 14 shows the serum antibody avidity of the Balb/c mice immunized with FH002C or Al001 combined with recombinant SARS-CoV-2 S protein, n=4 or 5, mean±SEM.
Fig. 15 shows the serum antibody avidity of the Syrian golden hamsters immunized with FH002C or Al001 combined with recombinant SARS-CoV-2 S protein, n=16, mean±SEM.
Fig. 16 shows the serum antibody subtypes of the Balb/c mice immunized with FH002C or Al001 combined with recombinant SARS-CoV-2 S protein, * p<0.05; ** p<0.01; ***p<0.001; **** p<0.0001; n=4 or 5, mean±SEM.
Fig. 17 shows the T cell response results of the Balb/c mice immunized with FH002C or Al001 combined with the recombinant SARS-CoV-2 S protein as detected by enzyme-linked immunospot method. Fig. 17A shows the captured image, and Fig. 17B shows the counting result of the captured image. *p<0.05; **p<0.01; ***p<0.001; ****p<0.0001; n=8, mean±SD.
Fig. 18 shows the specific antibody binding titer results of the Balb/C mice immunized with FH002C or Al001 combined with varicella-zoster virus gE protein, * p<0.05; ** p<0.01; *** p<0.001; ****p<0.0001; n=5, mean±SEM.
Fig. 19 shows the specific antibody binding titer results of the Balb/C mice immunized with FH002C or Freund's adjuvant combined with influenza virus HA protein, * p<0.05; **p<0.01; ^{∗∗∗} p<0.001; ****p<0.0001; n=10, mean±SEM. Note: "Victoria/WT" means wild-type HA protein of Victoria-lineage influenza virus without being treated with deglycosylase (Pngase), "Victoria/Pngase" means wild-type HA protein of Victoria-lineage influenza virus treated with Pngase.
Fig. 20 shows the neutralizing antibody titer results of the Balb/C mice immunized with FH002C or Al001 combined with rotavirus VP4 protein, n=5, mean±SEM.
Fig. 21 shows the neutralizing antibody titer results of the guinea pigs immunized with FH002C or Al001 combined with rotavirus VP4 protein, n=5, mean±SEM.

### Sequence information

The sequences referred to in the present application is described in the table below.

**Table 1: Sequence information**

| SEQ ID NO: | Sequence description |
|---|---|
| 1 | Amino acid sequence of wild-type S protein |
| | |
| | |
| 2 | Amino acid sequence of gE protein |
| | MGTVNKPVVGVLMGFGIITGTLRITNPVRASVLRYDDFHIDEDKLDTNSV YEPYYHSDHAESSWVNRGESSRKAYDHNSPYIWPRNDYDGFLENAHEH HGVYNQGRGIDSGERLMQPTQMSAQEDLGDDTGIHVIPTLNGDDRHKIV NVDQRQYGDVFKGDLNPKPQGQRLIEVSVEENHPFTLRAPIQRIYGVRYT ETWSFLPSLTCTGDAAPAIQHICLKHTTCFQDVVVDVDCAENTKEDQLAE ISYRFQGKKEADQPWIVVNTSTLFDELELDPPEIEPGVLKVLRTEKQYLGV YIWNMRGSDGTSTYATFLVTWKGDEKTRNPTPAVTPQPRGAEFHMWNY HSHVFSVGDTFSLAMHLQYKIHEAPFDLLLEWLYVPIDPTCQPMRLYSTC LYHPNAPQCLSHMNSGCTFTSPHLAQRVASTVYQNCEHADNYTAYCLGI SHMEPSFGLILHDGGTTLKFVDTPESLSGLYVFVVYFNGHVEAVAYTVVS TVDHFVNAIEERGFPPTAGQPPATTKPKEITPVNPGTSPLLRYAAWTGGL AAVVLLCLVIFLICTAKRMRVKAYRVDKSPYNQSMYYAGLPVDDFEDSE STDTEEEFGNAIGGSHGGSSYTVYIDKTR |
| 3 | Amino acid sequence of HA protein |
| | MKTIIALSHILCLVFAQKLPGNDNSTATLCLGHHAVPNGTIVKTITNDQ IEVTNATELVQNSSIGEICDSPHQILDGENCTLIDALLGDPQCDGFQNK KWDLFVERSKAYSNCYPYDVPDYASLRSLVASSGTLEFNNESFNWTG VTQNGTSSACIRRSNNSFFSRLNWLTHLNFKYPALNVTMPNNEQFDKL YIWGVHHPGTDKDQIFLYAQSSGRITVSTKRSQQAVIPNIGSRPRIRNIP SRISIYWTIVKPGDILLINSTGNLIAPRGYFKIRSGKSSIMRSDAPIGKCN SECITPNGSIPNDKPFQNVNRITYGACPRYVKQSTLKLATGMRNVPEK QTRGIFGAIAGFIENGWEGMVDGWYGFRHQNSEGRGQAADLKSTQA AIDQINGKLNRLIGKTNEKFHQIEKEFSEVEGRIQDLEKYVEDTKIDLW SYNAELLVALENQHTIDLTDSEMNKLFEKTKKQLRENAEDMGNGCFK |
| | IYHKCDNACIGSIRNGTYDHDVYRDEALNNRFQIKGVELKSGYKDWIL WISFAISCFLLCVALLGFIMWACQKGNIRCNICI |
| 4 | Amino acid sequence of recombinant S protein |
| | MFVFLVLLPLVSSQCVNLTTRTQLPPAYTNSFTRGVYYPDKVFRSSVL HSTQDLFLPFFSNVTWFHAIHVSGTNGTKRFDNPVLPFNDGVYFASTE KSNIIRGWIFGTTLDSKTQSLLIVNNATNVVIKVCEFQFCNDPFLGVYY HKNNKSWMESEFRVYSSANNCTFEYVSQPFLMDLEGKQGNFKNLREF VFKNIDGYFKIYSKHTPINLVRDLPQGFSALEPLVDLPIGINITRFQTLLA LHRSYLTPGDSSSGWTAGAAAYYVGYLQPRTFLLKYNENGTITDAVD CALDPLSETKCTLKSFTVEKGIYQTSNFRVQPTESIVRFPNITNLCPFGE VFNATRFASVYAWNRKRISNCVADYSVLYNSASFSTFKCYGVSPTKLN DLCFTNVYADSFVIRGDEVRQIAPGQTGKIADYNYKLPDDFTGCVIAW NSNNLDSKVGGNYNYLYRLFRKSNLKPFERDISTEIYQAGSTPCNGVE GFNCYFPLQSYGFQPTNGVGYQPYRVVVLSFELLHAPATVCGPKKSTN LVKNKCVNFNFNGLTGTGVLTESNKKFLPFQQFGRDIADTTDAVRDPQ TLEILDITPCSFGGVSVITPGTNTSNQVAVLYQDVNCTEVPVAIHADQL TPTWRVYSTGSNVFQTRAGCLIGAEHVNNSYECDIPIGAGICASYQTQT NSPGSASSVASQSIIAYTMSLGAENSVAYSNNSIAIPTNFTISVTTEILPV SMTKTSVDCTMYICGDSTECSNLLLQYGSFCTQLNRALTGIAVEQDKN TQEVFAQVKQIYKTPPIKDFGGFNFSQILPDPSKPSKRSFIEDLLFNKVT LADAGFIKQYGDCLGDIAARDLICAQKFNGLTVLPPLLTDEMIAQYTS ALLAGTITSGWTFGAGAALQIPFAMQMAYRFNGIGVTQNVLYENQKLI ANQFNSAIGKIQDSLSSTASALGKLQDVVNQNAQALNTLVKQLSSNFG AISSVLNDILSRLDKVEAEVQIDRLITGRLQSLQTYVTQQLIRAAEIRAS ANLAATKMSECVLGQSKRVDFCGKGYHLMSFPQSAPHGVVFLHVTY VPAQEKNFTTAPAICHDGKAHFPREGVFVSNGTHWFVTQRNFYEPQII TTDNTFVSGNCDVVIGIVNNTVYDPLQPELDSFKEELDKYFKNHTSPD VDLGDISGINASVVNIQKEIDRLNEVAKNLNESLIDLQELGKYEQGSGY IPEAPRDGQAYVRKDGEWVLLSTFLGSLEHHHHHHHH |

### Specific Models for Carrying Out the present invention

The present invention is described with reference to the following examples that are intended to illustrate the present invention (rather than limit the present invention), but those skilled in the art will understand that the following drawings and examples are only used to illustrate the present invention, rather than to limit the scope of the present invention. Various objects and advantages of the present invention will become apparent to those skilled in the art from the accompanying drawings and the following detailed description of the preferred embodiments.

The reagents used in the preparation examples were as follows:
Sodium risedronate (C₇H₁₀NNaO₇P₂), purchased from Hunan Huateng Pharmaceutical Co., Ltd.;
Anhydrous zinc chloride (ZnCh), purchased from Xilong Chemical Industry;
Aluminum chloride hexahydrate (AlCl₃·6H₂O), purchased from Xilong Chemical Industry;
Disodium hydrogen phosphate dodecahydrate (Na₂HPO₄·12H₂O), purchased from Xilong Chemical Industry;
Sodium hydroxide (NaOH), purchased from Xilong Chemical Industry.

### Preparation Example 1: Preparation of zinc aluminum risedronate adjuvant (FH002C)

### (1) Preparation of solutions:

According to the Zn/Al/risedronate molar concentration ratio of 1:0.1:0.22, 0.5 L of (47 mM zinc chloride+4.7 mM aluminum chloride) solution was prepared, which was defined as Solution A; 0.5 L of (10.3 mM risedronic acid + 55 mM sodium hydroxide + 63 mM disodium hydrogen phosphate) solution was prepared, which was defined as Solution B; and Solution A and Solution B were filtered through a 0.22 µm filter membrane for later use.

### (2) Preparation of zinc aluminum risedronate adjuvant FH002C suspension:

Zinc aluminum risedronate adjuvant was prepared by co-precipitation with the volume ratio of Solution A and Solution B being 1:1, that was, Solution B as prepared was added dropwise into Solution A at a volume ratio of 1:1 until Solution B was completely added to form a suspension. The suspension obtained was sterilized once at 121°C for 60 min, and the physical and chemical properties such as pH, particle size and particle shape after sterilization were measured.

Preparation Example 2: Preparation of zinc aluminum risedronate adjuvants (FH002C) with different ratios of zinc, aluminum and risedronate

### (1) Preparation of solutions:

With the molar concentration of Zn as 1, the designed Zn/Al/risedronate molar concentration ratios were 1:0.02:0.1, 1:0.02:0.15, 1:0.02:0.22, 1:0.02:0.33, 1:0.02:0.5, 1:0.02:1 and 1:0.05:0.1, 1:0.05:0.15, 1:0.05:0.22, 1:0.05:0.33, 1:0.05:0.5, 1:0.05:1 and 1:0.1:0.1, 1:0.1:0.15, 1:0.1:0.22, 1:0.1:0.33, 1:0.1:0.5, 1:0.1:1 and 1:0.15:0.1, 1:0.15:0.15, 1:0.15:0.22, 1:0.15:0.33, 1:0.15:0.5, 1:0.15:1 and 1:0.25:0.1, 1:0.25:0.15, 1:0.25:0.22, 1:0.25:0.33, 1:0.25:0.5, 1:0.25:1 and 1:0.5:0.1, 1:0.5:0.15, 1:0.5:0.22, 1:0.5:0.33, 1:0.5:0.5, 1:0.5:1; respectively, 0.5 L of(47 mM zinc chloride + 0.94, 2.35, 4.7, 7.05, 11.75, or 23.5 mM aluminum chloride) solution was prepared, which was defined as Solution A; 0.5 L of (4.7, 7.05, 10.3, 15.51, 23.5, or 47 mM risedronate + 35~250 mM sodium hydroxide + 63 mM disodium hydrogen phosphate) solution was prepared, which were defined as Solution B; Solution A and Solution B were filtered through a 0.22 µm filter membrane for later use.

### 2) Preparation of suspensions:

Zinc aluminum risedronate adjuvant was prepared by co-precipitation with the volume ratio of Solution A and Solution B being 1:1, that was, Solution B as prepared was added dropwise into Solution A at a volume ratio of 1:1 until Solution B was completely added to form a suspension. The suspension obtained was sterilized once at 121°C for 60 min.

### Preparation Example 3: Preparation of aluminum adjuvant Al001

According to the phosphate/Al molar concentration ratio of 0.15:1, 0.5 L of 124 mM aluminum chloride solution was prepared, which was defined as Solution A; 0.5 L of 18.6 mM disodium hydrogen phosphate solution was prepared, which was defined as Solution B, Solution B contained 140 mM sodium hydroxide, and was filtered through a 0.22 µm filter membrane for later use.

The preparation process of aluminum adjuvant Al001 suspension could refer to the preparation of FH002C suspension.

### Example 1: Determination of physical and chemical properties of zinc aluminum risedronate (FH002C) and aluminum adjuvant (A1001)

The FH002C suspension (Preparation Example 1) and Al001 suspension (Preparation Example 3) obtained were sterilized once at 121°C for 60 min, and their physical and chemical properties such as pH, particle size, particle shape, and zero charge point after sterilization were measured.

### (1) Observation of adjuvant appearance and particle morphology

After the zinc aluminum risedronate adjuvant FH002C was diluted 1-times with 100mM NaAc buffer solution and the aluminum adjuvant Al001 was diluted 1-times with normal saline, photos were taken with Mate30 5G mobile phone camera from China Huawei to record their adjuvant appearances; meanwhile, AE31E inverted biological microscope from China Motic was used to observe and photograph their particle morphology, in which the magnification of eyepiece and objective lens was 10x.

The aluminum adjuvant Al001 and the zinc-aluminum risedronate adjuvant FH002C were diluted 50-times and 100-times with deionized water, respectively, and then observed with JEM-2100 transmission electron microscope (TEM) from Japan Electronics. The specific steps were as follows: an adjuvant sample was dropped on a copper grid, and allowed to be absorbed for 10 min, after the residual liquid being wiped off with filter paper, the sample was send into the sample chamber of the transmission electron microscope to observe morphology, and photographed.

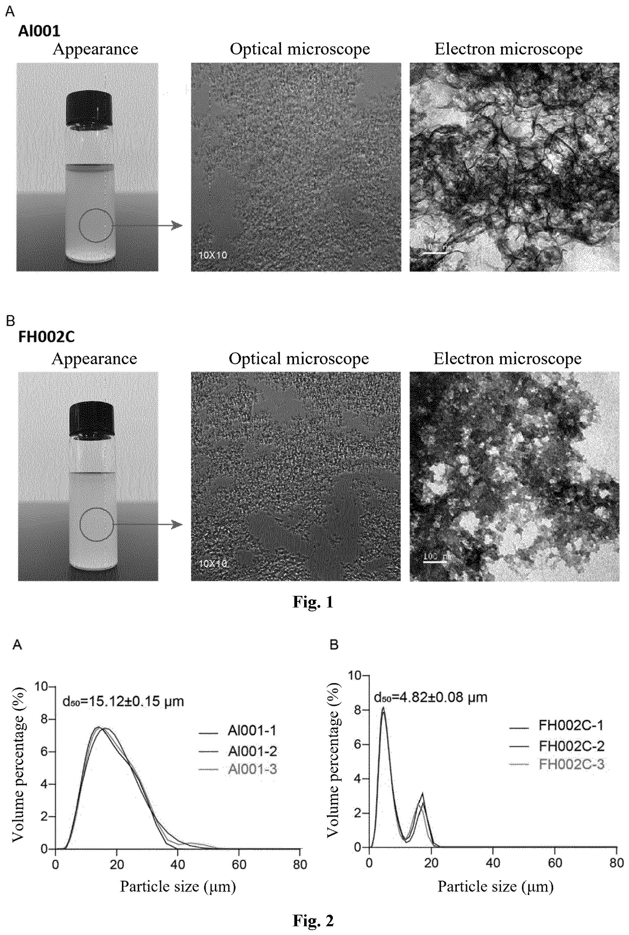

Experimental results: As shown in Fig. 1, the zinc-aluminum risedronate adjuvant FH002C and aluminum adjuvant Al001 were both milky white suspensions; under the optical microscope, both were amorphous clusters; under the electron microscope, FH002C showed a irregular sheet-like structure, while Al001 adjuvant showed a clear filamentous structure.

### (2) pH measurement

A test sample was taken, placed at room temperature for at least 30 minutes, and measured with a Sartorius acidity meter.

Standard buffer solution (pH 7.00), standard buffer solution (pH 4.01), and standard buffer solution (pH 10.01) were selected and used to calibrate the instrument according to the instruction manual.

"Mode" (switch) key could be pressed to switch between pH mode and mV mode. Usually, the mode was set at pH mode to determine solution pH value.

"SETUP" key was pressed, the display displayed "Clear buffer", "ENTER" key was pressed to confirm, and clear the previous calibration data.

"SETUP" key was pressed until the display showed the buffer solution group "4.01, 7.00, 10.01", then "ENTER" key was pressed to confirm.

Electrode was taken out from the electrode storage solution, and rinsed fully with deionized water, and the surface water was dried with filter paper after rinsing (note: the electrode should not be wiped).

The electrode was dipped into the first buffer solution (pH 7.00) until the value was stable and "S" appearred, "STANDARDIZE" key was pressed to automatically calibrate the instrument. After successful calibration, "7.00" and electrode slope would be displayed.

The electrode was taken out from the first buffer solution, and fully rinsed with deionized water, and the electrode was dipped into the second buffer solution (pH 4.01) in turn until the value was stable and "S" appeared, "STANDARDIZE" key was pressed to automatically calibrate the instrument. After successful calibration, "4.01 7.00" and message "Slope" would be displayed. "Slope" showed the measured electrode slope value, which was acceptable in the range of 90-105%.

If there was a relatively large deviation from the theoretical value, an error message (Err) would be displayed, the electrode should be cleaned, and the above steps should be repeated for calibration.

The above operations were repeated to complete the third point (pH 10.01) calibration.

After calibration, the electrode was rinsed thoroughly with deionized water, then gently blotted to dry with filter paper. A test solution was shaken well, the glass electrode was dipped into the test solution, and a reading was taken when the pH value did not change more than ±0.05 within 1 minute.

The test solution was shaken and measured again, and the difference between the two pH values should not exceed 0.1. The average value of the two readings was taken as the pH value of the test solution.

Experimental results: The pH of the Al001 adjuvant was 8.00-8.40 before sterilization, and 6.50-6.90 after sterilization; the pH of FH002C adjuvant was 7.00-7.50 before sterilization, and 5.80-6.30 after sterilization.

### (3) Determination of particle size

Beckman LS 13320 laser particle size tester was turned on and the preheating was performed for 15 min.

The instrument control software and the closed compartment of sample cell were opened, the sample cell was taken out from the sample tank, and 12 mL of purified water was added.

The sample cell was placed on the sample holder and the door was closed.

After opening the LS13320 software, "OK" was clicked in the pop-up window to perform the instrument self-test. "run" on the menu bar was clicked and "Use Optical Module" was selected to open the detection module. "control" on the menu bar was clicked and "stirrer on" was selected to start the automatic stirrer built into the sample cell. "start cycle" was clicked, and "Measure Offsets", "Align", "Measure Background" were selected in turn, and finally "start" was clicked, and "OK" in the pop-up dialog box was clicked to start the calibration of the blank background.

The sample cell was taken out, a certain amount of standard sample (included with the instrument) was added, "start cycle" was clicked, and "Measure Loading", "Enter Sample Info", "Enter run setting", "start runs" were selected in turn, and finally "start" was clicked, the name of the standard sample was entered in the pop-up dialog box, and "OK" was clicked when the "Obscuration" parameter in the software was 8% to 12% to test the standard sample.

In order to ensure the accuracy and reliability of the experimental data, it was necessary to calibrate the size of the blank background and the test standard sample before each start-up for measurement.

The closed compartment of the sample cell was opened and the sample cell was taken out.

The aqueous solution containing the standard sample in the sample cell was discarded, and deionized water was poured into the sample cell to clean the sample cell 3 times.

After cleaning, 12 mL of deionized water was added, the sample cell was placed on the sample tank, and the door was closed.

"start cycle" was clicked, and "Measure Offsets", "Align", "Measure Background" were selected in turn, and finally "start" was clicked, and "OK" in the pop-up dialog box was clicked to start the calibration of the blank background.

The sample cell was taken out, a certain volume of test sample was added, the sample test door was opened, the sample cell was placed on the sample tank, and the door was closed.

"start cycle" was clicked, and "Enter Sample Info", "Enter run setting", "start runs" were selected in turn, and finally "start" was clicked, the sample name was entered in the pop-up dialog box, "OK" was clicked when the "Obscuration" parameter in the software was 8% to 12% to record the particle size of the sample as measured; three repeated measurements were performed on the aluminum adjuvant Al001 and zinc aluminum risedronate FH002C, respectively.

Experimental results: As shown in Fig. 2, the Al001 showed a unimodal particle distribution, in which the size was between 0.4-60 µm and mainly distributed at 15-16 µm (Fig. 2A); the zinc-aluminum risedronate adjuvant FH002C showed a bimodal particle distribution, in which the size was between 0.4-25 µm, and the particle size of the main peak was around 5.0 µm (Fig. 2B).

### (4) Detection of PZC (point of zero charge)

### Instrument for detection: Nanobrook Omni (Brookhaven)

Experimental operation: The power was turned on, and the multi-angle granularity and high-sensitivity Zeta potential meter were preheated for 30 minutes.

By using 36%-38% hydrochloric acid and 10 M NaOH, the Al001 adjuvant was adjusted to have a pH of 6.5, 7.0, 7.5, 8.0, 8.5, and the FH002C adjuvant was adjusted to have a pH of 3.0, 4.0, 5.0, 6.0, 7.0.

Passivation of electrode: 3-4 mL of the adjuvant was to the sample tube. After the electrode was inserted, the cycle was set to 50 in the SOP, and the instrument was run to passivate the electrode.

Sample detection: The electrode was taken out, then rinsed at the lower end with deionized water, the corresponding sample was added, "instrument parameters" in the SOP were clicked, "square polystyrene cell" in "cell type" was selected, "BI-SREL(1250 µL)" in "electrode assembly" was selected, then "advanced settings" was clicked, "equilibration time" was set to 120 seconds, "inter-cycle delay" was set to 1 seconds, "measurements" in "time dependent" was set to 3, "time interval" was set to 0, "liquid" was clicked, the pH was set to the pH corresponding to each sample, "model" in "Data Analysis" was clicked, "smoluchowski" was selected, and finally "save" was clicked, "OK" clicked to complete the parameter setting.

Instrument running: After fully shaking the adjuvants with different pH values, 2 mL was pipetted and transferred into the plastic sample tank in turn, the electrode was inserted, then it was placed into the sample tank, the instrument was connected to the sample tank to collect data, and "start" was clicked to start the measurement. Then the next sample test was performed.

Data processing: The corresponding Zeta potentials at different pH values were obtained, the software that came with the instrument was run to get the PZC values, and then GraphPad Prism 8.0.2 (manufacturer: GraphPad Software, LLC) was used to draw the diagrams.

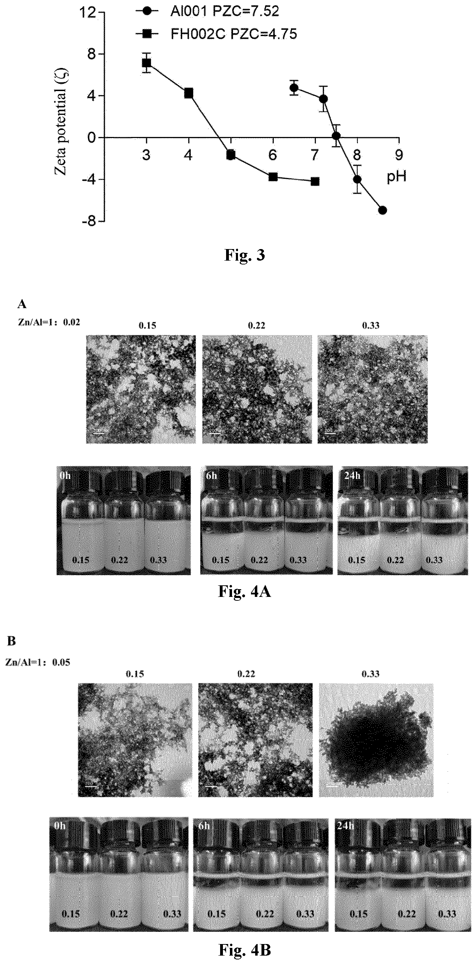

Experimental results: As shown in Fig. 3, the PZC of the aluminum adjuvant Al001 was 7.52; the PZC of the zinc-aluminum risedronate adjuvant FH002C was 4.75.

### (5) Precipitation rate of Zn/Al/risedronate in FH002C

Experimental method: The contents of zinc and aluminum in the free supernatant were detected by atomic absorption spectrometer (Shimadzu, AA6300C (PIN 206-52430)), and risedronate was detected by ultraviolet spectrophotometer (Beckman, DU800). The details were as follows:
Determination of zinc element content by flame method using atomic absorption spectrometer:
Preparation of standard curve: Zinc single-element standard solution (Beijing General Research Institute for Nonferrous Metals, GSB 04-1761-2004) was diluted with 0.2% nitric acid to obtain 0, 500, 1000, 1500, 2000 ng/mL zinc standard solutions.
Preparation of test solution: The sample was diluted with 0.2% nitric acid solution until the concentration was within the range of the standard curve (Abs reading value was between 0.2 and 0.8), and shaking and mixing with a vortex mixer were performed before each step of dilution.
Log-in to WizAArd: WizzAArd icon was double-clicked to open the software, the "Measurement" icon in the "Operation" column was clicked, "Admin" was entered in the "Login ID" column, and <OK> was clicked without a password.
Wizard selection: "Element Selection" was selected, and <OK> was clicked.
Element selection: "Select Element" option on the "Element Selection" page was selected, "Zn" on the "Loading Parameters" page was selected, [Flame Continuous] measurement method was selected, "Use ASC" option was not ticked, [Common Lamp] was selected, and <OK> was clicked. "Calibration Curve Settings" and "Edit Parameters" pages were not specially set, and <OK> was clicked. On the "Not Connected Instrument/Send Parameters" page, <Connect/Send Parameters> was clicked.

On the "Instrument Initialization" page, the options for ASC inspection and GFA inspection of the graphite furnace method did not need to be checked; the "Check Gas" dialog box appeared, <No> was selected; "Now the safety device can be checked, will the burner pressure monitor be checked?" dialog box appeared, and <Yes> was selected; "Please supply air, click the OK button after supplying air, air discharge" dialog box appeared, and <OK> was selected; "Check NO₂" appeared, and <No> was selected; "NO₂-C₂H₂ cannot be used" dialog box appeared, and <OK> was selected; "Elevate the waste liquid probe above the liquid level" appeared, operation was carried out by following the prompts and enough ultrapure water was added to the waste liquid tank until the water flowed out from the waste liquid tube to prevent backfiring; "Move the waste liquid probe below the liquid surface" appeared, operation was carried out by following the prompts and the opening of the waste liquid tank was closed. After all items were checked, <OK> was clicked.

All items on the "Instrument Inspection List for Flame Analysis" page were checked and ticked, and <OK> was clicked.

On the "Optical Parameters" page, the wavelength was set at [213.86 nm], the slit width was set at [0.7], the lighting method was set as [emission], [lamp position setting] was set to ensure that the actual position of the Zn hollow cathode lamp was the same as the set position, and [Lighting] was selected, when "Ready" was displayed at the bottom of the screen, <OK> was clicked.

On the "Spectral Line Search" page, when both "Spectral Line Search" and "Beam Balance" displayed [OK], [Close] was clicked; after returning to the "Optical Parameters" page, <Next> was clicked. On the "Atomizer/Gas Flow Settings" page, no change was made, and <Finish> was clicked.

Burner origin position adjustment: [Instrument]→[Maintenance]→[Burner Origin Position Adjustment] were selected in turn, the front and rear position knobs and the wrench on the burner were adjusted to control the position of the burner, the AA-6300C standard card was used to observe the position of the emitted light, the scale on the card was used to make the light passing through the entire burner slit on the same horizontal line. After the adjustment, the Abs value became stable; the vertical height of the burner was adjusted through the computer by clicking <move up> or <move down>, coarse adjustment first and then fine adjustment, so that the Abs value was half of the maximum reading (it ensured that 1/2 of the maximum light intensity passed through the burner), <Origin Memory> was selected, the current page was closed, and a safety cover was placed above the burner.

In the menu bar, [Parameter]→[Edit Parameter]→ were selected, and the lighting method was changed to <BGC-D2>. "Line Search" was performed again, and when both "Line Search" and "Beam Balance" displayed [OK], <Close> was clicked.

Ignition: After ensuring that C₂H₂ was turned on and the pressure met the requirements, the PURGE and IGNITE keys on the host were pressed at the same time until ignited.

Automatic zero adjustment: Before formal detection of samples, [auto zero adjustment] was selected to remove residual impurities in the burner.

On the MRT worksheet, the blank group (BLK), standard substance (STD), and sample to be tested (UNK) were set, the theoretical concentration of the standard substance and the name of the sample were entered, and the sample was manually loaded through the sample loading tube extended from the atomizer. The sample volume at each time was at least 1mL, and [Start] was selected to perform detection.

Data processing: Scatter diagram was made in Excel based on the Abs and concentrations of the standard, a trend line was added, the formula and the correlation coefficient R² were displayed, which showed that R² ≥ 99% and proved that the correlation was good, and could be used to calculate the sample concentration.

Determination of aluminum element content by graphite method with atomic absorption spectrometer:
Preparation of standard curve: Aluminum single-element standard solution (National Nonferrous Metals and Electronic Materials Analysis and Testing Center, GNM-SAl-002-2013) was diluted with 2% nitric acid to obtain 0, 20, 50, 100, 150, 200 ng/mL aluminum standard solutions.
Preparation of test solution: The sample was diluted with 0.2% until the concentration was within the range of the standard curve (Abs reading was between 0.2 and 0.8).
Log-in to WizAArd: WizzAArd icon was double-click to open the software, the "Measurement" icon in the "Operation" column was clicked, "Admin" was entered in the "Login ID" column, and <OK> was clicked without a password. Wizard Selection: "Element Selection" was selected, and <OK> was clicked.
Element selection: "Select Element" option on the "Element Selection" page was selected, "Al" on the "Loading Parameters" page was selected, "Use ASC" option was ticked, [Common Light] was selected, and <Next> was clicked. "Calibration Curve Settings" and "Edit Parameters" pages did not require special settings, and <OK> was clicked. The power switch wrench (HEAT) of GFA-EX7i was turned, and <Connect/Send Parameters> on the "Connect to Host/Send Parameters" page was clicked. When the dialog box "The instrument is not connected, will it be connected?" appeared, <Yes> was clicked.

On the "Instrument Initialization" page, the options for C₂H₂ valve, burner, gas, flame monitor, burner, and waste liquid probe of the flame method did not need to be checked, and <NO> was clicked. After all items were checked, <OK> was clicked. Bypassing flame analysis: When the "Will flame analysis be performed?" dialog box appeared, <No> was clicked.

On the "Optical Parameters" tab page, the wavelength was set at [396.2 nm], the slit width was set at [0.7 nm], the lighting method was set as [emission], [lamp position setting] was set to ensure that the aluminum lamp was at the set position, [lighting] option was ticked, and when [Ready] was displayed in the lower right corner of the screen, <OK> was clicked.

"Spectral Line Search" page: When both "Spectral Line Search" and "Beam Balance" displayed [OK], <Close> was clicked; after returning to the "Optical Parameters" page, <Next> was clicked.

On the "Graphite Furnace Program" page, [7] for "Maximum Phase Number", [6] for "Sampling Phase Number", and [Unknown] for "Graphite Tube Type" were selected, and <Finish> was clicked.

Graphite furnace atomizer origin position adjustment: [Instrument], [Maintenance], [Graphite furnace origin position adjustment] were selected in turn; after the atomizer position reading became stable, the graphite furnace atomizer was moved by using the manual front and rear position knobs until the Abs reading reached its maximum, the WizAArd software was used to adjust the up and down position of the atomizer, first [Fast] adjustment and then [Slow] adjustment, until the reading reached its maximum, and <Origin Memorization> was clicked.

Checking ASC nozzle position: [Instrument]→[Graphite Furnace Nozzle Position] on the menu bar were selected in turn; following the computer prompts, the sample-sucking nozzle was firstly moved to the ASC turntable, and then moved to the graphite furnace atomizer; according to the prompts of WizAArd software, the arm guide screw was firstly loosened, <Move Down> was clicked to move the sample-sucking nozzle down, and when it was close to the injection hole of the graphite furnace, the injection position adjustment knob of the ASC workbench was adjusted, and the position of the sample-sucking nozzle was adjusted horizontally from front to back, left and right, until it was at the center of the injection hole. <Move Up> and <Move Down> were used to adjust the sample loading position, first rough adjustment and then fine adjustment, the observation mirror equipped on ASC was used to observe the position of the sample-sucking nozzle in the injection hole, and the position was optimized at 1/3 of the distance between the sample-sucking nozzle and the bottom of the injection hole. <OK> was clicked to save the current position and close the current page.

Repeatedly checking ASC nozzle position: The knob was tightened, [Instrument]→[Graphite Furnace Nozzle Position] on the menu bar were selected in turn; after the sample-sucking nozzle was moved to the atomizer, [Direction Moving Tube] was select; if the sample-sucking nozzle was at the center of the injection hole, there was no need to adjust it; if the position of the sample-sucking nozzle was offset, it was adjusted to the center of the injection hole, and <Cancel> was selected until the sample-sucking nozzle was at the center of the injection hole.

In the menu bar, [Parameter]→[Edit Parameter]→ were selected, and the lighting method was changed to <BGC-D2>. "Line Search" was performed again, and when both "Line Search" and "Beam Balance" displayed [OK], <Close> was clicked. Cleaning: Before formally testing samples, <Cleaning> was selected to remove residual impurities in the graphite tube.

On the MRT worksheet, the positions and sample volumes (10 µL) of the blank group (BLK), standard substance (STD), and sample to be tested (UNK) were set in turn, the theoretical concentration of the standard substance and the sample name of the sample to be tested were entered, and <Start> wsa selected to carry out the detection.

Result processing: According to the Abs values and theoretical concentrations of the standard, a scatter diagram was made in excel, a trend line was added, and the formula and the correlation coefficient R² were displayed, in which R²≥99% proved that the correlation was good, and could be used to calculate the sample concentration.

The risedronate content of the FH002C supernatant was determined by a UV spectrophotometer:
Power-on and preheating: The main power switch on the right rear side of the instrument was turned on, general preheating was performed for 10-20 minutes.
Performing option measurement according to requirements: "Fixed Wavelength" (fixed wavelength measurement) in the optional column of the first line in the upper left corner of the screen was selected to perform the measurement.
Fixed Wavelength (fixed wavelength measurement): "Edit Method" icon was clicked to display the wavelength setting page, "262 nm wavelength" in the "Number of wavelengths" column was selected, and "OK" was clicked after setting. The sample in the blank tube was loaded into a clean cuvette and placed into a colorimetric rack, the "BLK" icon was clicked to deduct the blank, then the samples were added separately, and "Read" was clicked.

Experimental results: Based on the obtained standard curve, the zinc ions, aluminum ions and risedronate in the supernatant of FH002C were calculated respectively, according to the formula: (total content of each component - content of each component in the supernatant)/total concentration of each component *100%, that was, the precipitation rate of each component. Through the calculation, in the zinc aluminum risedronate adjuvant FH002C, the precipitation rate of zinc was greater than 99.0%, and the precipitation rates of aluminum and risedronate were greater than 99.9% (Table 2).

**Table 2: Precipitation rates of zinc ions, aluminum ions and risedronate in FH002C**

| Adjuvant name | Precipitation rate (%) | | |
|---|---|---|---|
| | Zn²⁺ | Al³⁺ | Risedronate |
| FH002C | >99.0% | >99.9% | >99.9% |

### (6) Determination of adsorption rate

The recombinant SARS-CoV-2 S protein was designed and prepared as follows. Briefly, the trimerization domain of T4 phage fibritin protein was fused to the C-terminal of the full-length extracellular segment of the wild-type S protein (SEQ ID NO:1) of SARS-CoV-2, the furin cleavage site was removed, and a 6*His tag was fused at the C-terminal to design a recombinant SARS-CoV-2 S protein (recombinant S protein, SEQ ID NO:4). The recombinant S protein was expressed by Chinese hamster ovarian cancer cell (CHO) expression system (purchased from Thermo Scientific, A29133), and purified by nickel-agarose column (Cytiva, 17-5318-03), so as to obtain a recombinant S protein with a purity of greater than 95%. The purified recombinant S protein was quantified by the BCA method (Pierce^{™} BCA Protein Assay Kit, Thermo Scientific, 23227). The recombinant S protein was diluted to 400 µg/mL, mixed with the FH002C adjuvant, recombinant S protein:adjuvant = 1:1 (volume ratio), the mixed sample preparation was shaken and allowed to stand at 4°C for adsorption. Sampling was performed at each of time points separately. Shaking well was performed before sampling, 500 µL/time; the sample as taken out was centrifuged at 13,000 rpm/min for 5 min, then the supernatant was taken, and then the sample supernatant was diluted by the corresponding fold according to the pre-experiment results for later use.

Drawing standard curve: The recombinant S protein stock solution was used as standard and subjected to gradient dilution, and the sample diluent SD-1 was used as dilution buffer, the standard was diluted to a series of concentrations as specified in EP tube: 50 ng/mL, 25 ng/mL, 12.5 ng/mL, 6.25 ng/mL, 3.12 ng/mL, 1.56 ng/mL, for later use.

### Experiment procedure:

(1) Coating plate: 1x CB9.6 coating buffer was used to dilute 36H6 monoclonal antibody (self-made in the laboratory, 36H6 monoclonal antibody was an antibody that recognized the RBD epitope on SARS-CoV-2 S protein, and prepared by hybridoma technology, and the method referred to *Li, et al. Emerging Microbes Infection. 2020*) to 1 µg/mL, then added at 100 µL/well to a polystyrene 96-well plate, and the coating was performed overnight at 4°C.
(2) Blocking: The coating solution in the wells was discarded, the plate was washed once with PBST washing solution, spin-dried, added with blocking solution-1, 200 µL/well, and the blocking was performed at 25°C for 4 h.
(3) The blocking solution in the wells was discarded, the plate was washed once with PBST, and spin-dried, the diluted standard and the sample supernatant were added to the corresponding 96-well plate, and incubated at 25°C for 1 h.
(4) Adding enzyme-labeled antibody (85F7-HRP) (self-made in the laboratory): The liquid in the wells was discarded, the plate was washed 5 times with PBST, spin-dried, and added with the enzyme-labeled antibody (85F7-HRP, 1:5000 (V:V) diluted with ED-11 as enzyme dilute solution) at 100 µL/well, and the incubation was performed at 25°C for 1 h.
(5) Color development: The enzyme-labeled antibody in the wells was discarded, the plate was washed 5 times with PBST, spin-dried, added with 100 µL/well of the mixed color-developing solutions A and B in equal volume, and the reaction was performed at 25°C for 10 min.
(6) Stopping: 50 µL/well of 2 M sulfuric acid stopping solution was added to stop the reaction.
(7) Plate reading: The detection dual wavelengths were set at 450 nm and 630 nm on the microplate reader, and the OD value of each reaction well was measured.

Calculation of FH002C adjuvant adsorption rate: The OD reading value of the diluted sample well was brought into the standard curve to obtain the corresponding protein concentration of the sample well, and then it was multiplied by the corresponding dilution factor, thereby obtaining the protein concentration of the supernatant of the original sample. Adjuvant adsorption rate = (total protein concentration of original sample - protein concentration of supernatant) / total protein concentration of original sample * 100%.

The experimental results were shown in Table 3. According to the calculation formula of adsorption rate, when the vaccine preparation of FH002C combined with recombinant S protein was adsorbed for 30 hours, the adjuvant adsorption rate could reach more than 95%; and when it was allowed to stand for 70 days at 4°C, the protein adsorption rate in the vaccine preparation was always above 95% (96.5% to 99.9%).

**Table 3. Adsorption rate of FH002C adjuvant to recombinant SARS-CoV-2 S protein**

| Adjuvan t name | Protein molecule | | Protein adsorption rate (%) | | | | | | | |
|---|---|---|---|---|---|---|---|---|---|---|
| | | 4h | 18h | 24h | 30 h | 72 h | 7d | 21d | 30 d | 70d |
| FH002C | Recombinan t S protein | 92.1 | 93.4 | 93.8 | 95.0 | 98.1 | 99.9 | 98.9 | 96.5 | 99.9 |

### Example 2: Determination of physical and chemical properties of zinc aluminum risedronate adjuvant (FH002C) with different ratios of zinc, aluminum and risedronate

The FH002C suspension (Preparation Example 2) and Al001 suspension (Preparation Example 3) obtained were sterilized once at 121°C for 60 min, and their physical and chemical properties such as pH, adsorption rate, metal precipitation rate, appearance and particle morphology were determined.

### (1) Determination of pH

The method for determination of pH was the same as the pH determination process in Example 1.

Experimental results: The pH values of FH002C adjuvant with different ratios of zinc, aluminum and risedronate were 7.20-7.90 before sterilization, and 6.00-6.60 after sterilization.

### (2) Determination of adsorption rate

Drawing standard curve for BSA standard: 100 mM NaAc was used as the dilution buffer, the BSA standard (2 mg/mL) was serially diluted, the absorbance at 280 nm was measured with a UV spectrophotometer, and the standard curve was drawn.

Preparation of BSA: 100 mM NaAc was used as the dilution buffer, and a certain amount of BSA sample was weighed and diluted into an EP tube to reach a final concentration of 1 mg/mL.

Mixing of BSA and adjuvant: After shaking the adjuvant, mixing was performed according to 1 mg/mL BSA:adjuvant = 1:1 (volume ratio), absorption was carried out at room temperature for 1 h, shaking was performed 5 times during the period, after centrifugation (13000 rpm/min, 3min), the supernatant was taken for later use.

Determination of adsorption rate: UV spectrophotometer was used to directly measure the absorbance value of the supernatant at 280 nm, so that the reading value was between 0.2-0.8. Calculation of adsorption rate: the OD reading value of the diluted sample well was brought into the standard curve to obtain the corresponding protein concentration of the sample well, and then it was multiplied by the corresponding dilution factor to obtain the protein concentration of the supernatant of the original sample. Adjuvant adsorption rate = (total protein concentration of original sample - protein concentration of supernatant) / total protein concentration of original sample * 100%.

The experimental results were shown in Table 4: According to the calculation formula of adsorption rate, the adsorption rates of FH002C adjuvant with different ratios of zinc, aluminum and risedronate were between 39% and 96%. Taking 75% as the standard, the adjuvants with an adsorption rate of greater than 75% were selected to observe the metal precipitation rate.

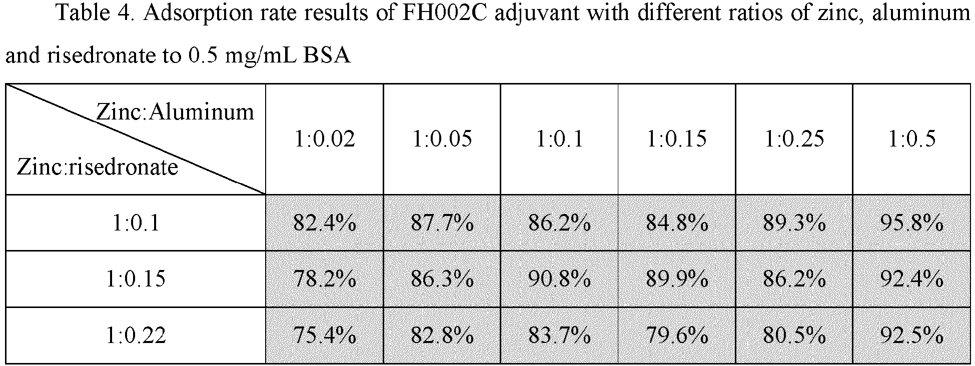

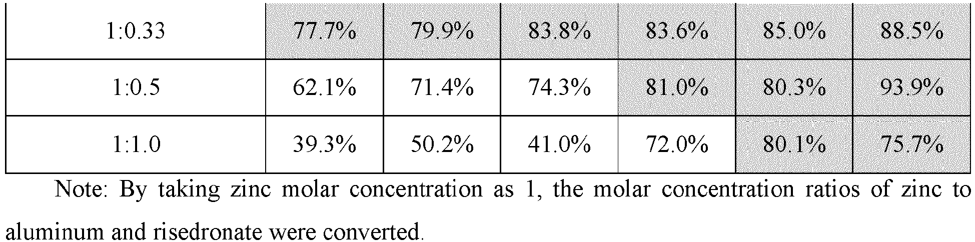

### (3) Detection of metal precipitation rate

Preparation of standard solution of mixed metal elements: In a 12 mL centrifuge tube, a standard solution with four metal single elements was diluted with 5% nitric acid to the highest concentration point of the calibration curve to obtain STD7 solution. Then it was diluted stepwise with 1% nitric acid (6 mL of hydrochloric acid was added to 6 mL of the previous standard working solution) to obtain STD6 to STD1 solutions in turn, and the final volume of each mixed standard solution was 6 mL to ensure that the sample was completely loaded.

**Table 5. Standard curves of mixed metals**

| | STD1 (ppm) | STD2 (ppm) | STD3 (ppm) | STD4 (ppm) | STD5 (ppm) | STD6 (ppm) | STD7 (ppm) |
|---|---|---|---|---|---|---|---|
| Zinc | 1.25 | 2.5 | 5 | 10 | 20 | 40 | 80 |
| Aluminum | 0.625 | 1.25 | 2.5 | 5 | 10 | 20 | 40 |
| Magnesium | 0.625 | 1.25 | 2.5 | 5 | 10 | 20 | 40 |
| Calcium | 0.625 | 1.25 | 2.5 | 5 | 10 | 20 | 40 |

| | | | | | | | |
|---|---|---|---|---|---|---|---|
| Note: The unit "ppm" in the table was equivalent to "µg/mL". | | | | | | | |

Preparation of test solution (at least 4 mL): The test sample was taken and equilibrated to room temperature, mixed thoroughly with a vortex mixer, the test sample was precisely sampled and diluted to an appropriate fold (200-fold was recommended for determination of calcium, magnesium and zinc contents, and 50-fold was recommended for determination of aluminum content) with 5% nitric acid solution, mixed well and allowed to stand for 1 hour, which was the test sample solution.

Preparation of blank control solution: 5% nitric acid solution was taken as blank control.

Measurement: Argon gas bottle (adjusted to have an air pressure of about 0.6 MPa) was opened; Equilibration with blowing gas: the ICP-OES instrument and software were turned on, "Purge Gas Flow" in the "Dashboard" of the software interface was selected, "Normal" model in the drop-down option was clicked. The instrument was equilibrated for at least 1 hour in this state. When equilibrating, "Torch compartment", "Plasma Gas Pressure", "Purge Gas Pressure", "Drain Flow", "Exhaust Flow" and "Optics Temperature" on the software interface were observed and should turn green. The water tank was opened; after waiting for about 2 minutes, "Detector Water Flow" and "Detector Temperature" in the instrument status bar should turn green. The pipeline of the instrument was connected and cleaned: the catheter inlet was placed in ultrapure water, the catheter was correctly connected to the peristaltic pump, "Pump Speed" in the selection interface was changed to 50, "Apply" was clicked, and it was observed whether liquid could normally flowed out the catheter outlet. After cleaning for 1-2 minutes, ignition could be carried out. Ignition: "Dashboard" in the software interface was selected, the middle blue icon "Get Ready" at the top of the operation interface was clicked, "OK" in a pop-up window was clicked, after waiting for about 2-5 minutes, the ignition was successful. If the first ignition was not recognized as normal, the ignition operation was performed again after the instrument was completely self-cleaned. The x and y values in the "Carbon line" in the log were observed. The absolute values of both should be less than 10, respectively. Establishment of LabBook: "LabBooks" on the right menu bar was clicked, the name of the experiment to be done was entered in the "Name" column (experiment date + experiment content + operator), "Create a new LabBook from an exsiting LabBook" was selected, "Risedronate Sodium- Zinc -Aluminum adjuvant Method Template" was opened, "Create LabBook" at the bottom of the interface was clicked, that was, the LabBook for the current experiment in the zinc aluminum risedronate adjuvant template was successfully established. Parameter selection: In the newly established template interface, the right side "Analytes" column was checked for the elements to be measured and their detection wavelengths, Zn (213.856nm), Al (396.152 nm), Ca (396.847 nm), Mg (279.553nm). In the "Measure Modes" column, it was checked whether the observation direction was "Radial". Establishment of sample loading sequence: "Sample list" in the right column was selected, the sample information and loading position were input in the sequence and saved, the green triangle icon "Start" in the upper left corner was clicked, and then the green triangle icon in the lower left corner was clicked to start running the sequence. When measuring each sample, the catheter was placed into the centrifuge tube. After each injection was measured, the residual droplets on the catheter should be wiped off with dust-free paper (or filter paper) before the system prompt that it should be placed into "wash" for cleaning. When the catheter was transferred from the "wash" into the next sample, the droplets on the catheter should wiped off as well. Calculation of results: Taking the concentration of the mixed standard solution as the abscissa, 4 standard curves were drawn with the corresponding peak areas as the ordinate, and a linear regression equation was drawn up. The peak area of each single element corresponding to the diluted test sample solution was substituted into the regression equation, and multiplied by the dilution factor, to obtain the metal element concentration (ppm) of the test sample solution.

Experimental results: As shown in Tables 6, 7 and 8, Zn basically existed in the precipitate, Mg and Ca partially existed in the precipitate, and partly existed in the supernatant. According to the precipitation rate results of Mg and Ca, the part marked gray was selected to observe the adjuvant appearance and particle morphology.

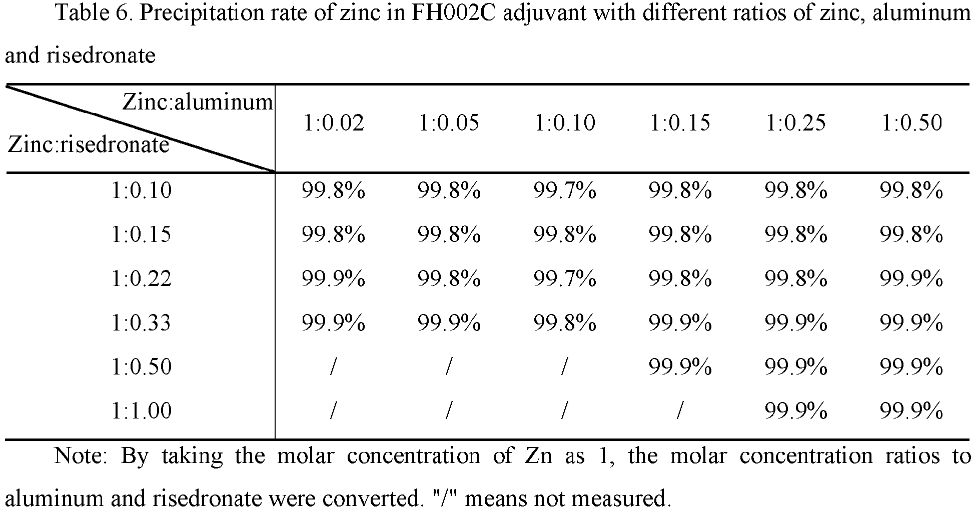

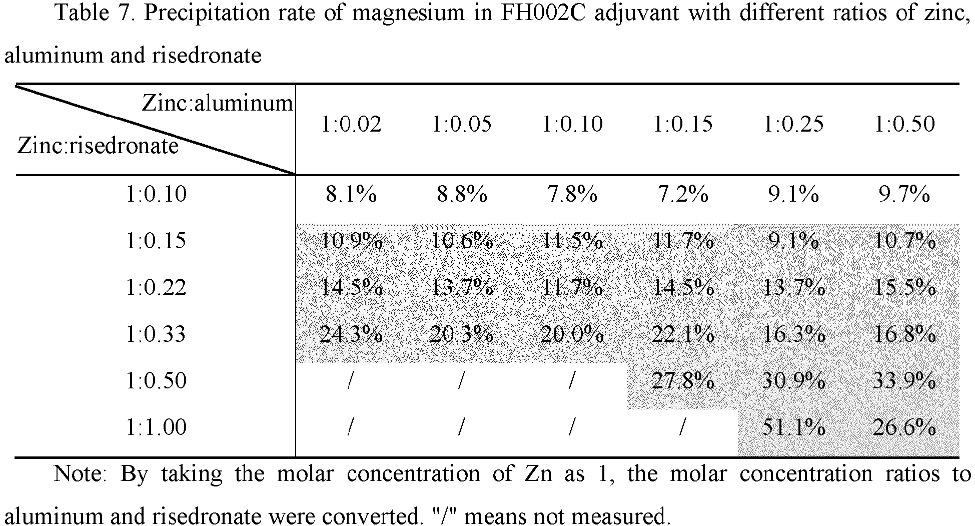

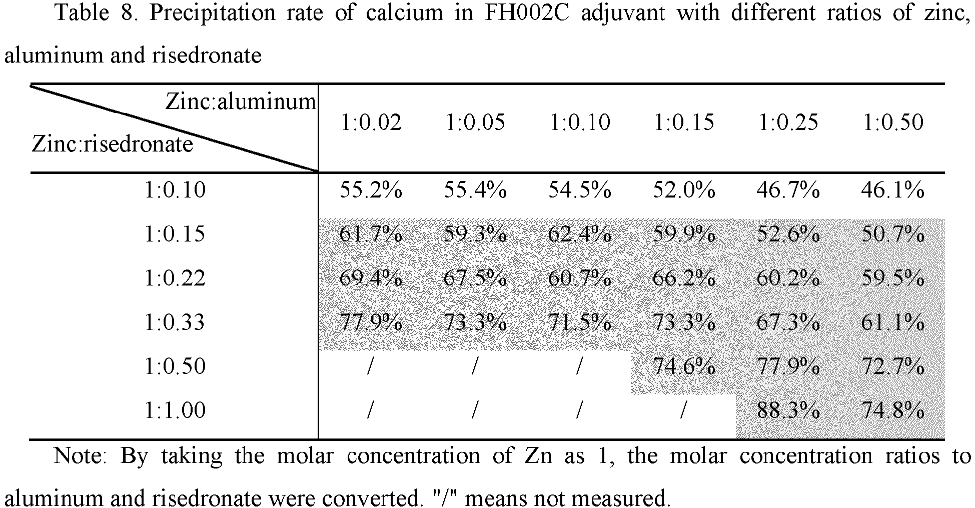

### (4) Observation of adjuvant appearance and particle morphology

The observation of the adjuvant appearance and particle morphology were performed by referring to the detection process in Example 1.

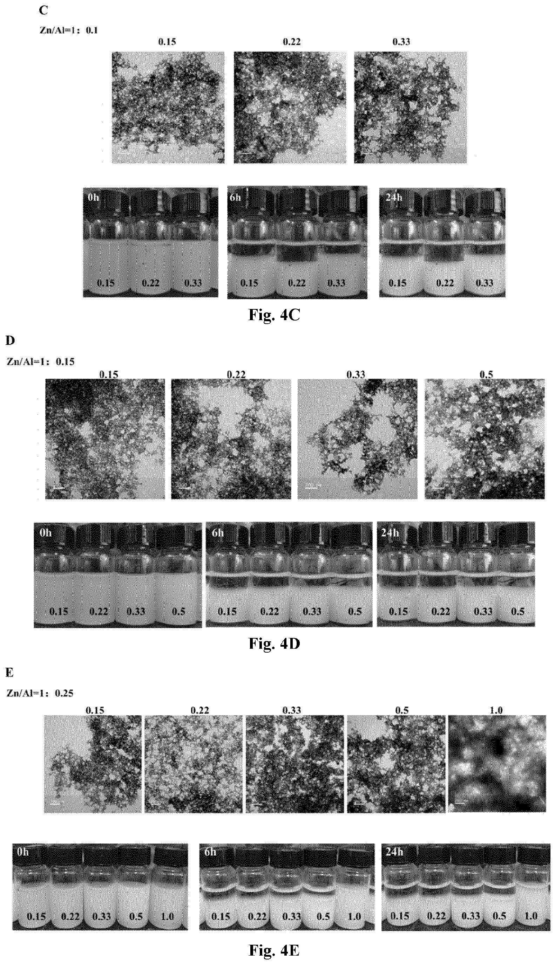

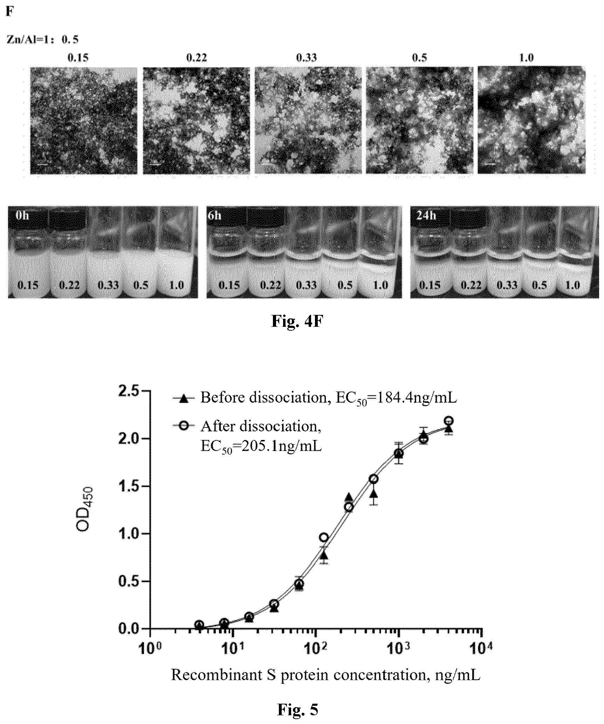

Experimental results: As shown in Fig. 4A to Fig. 4F, zinc-aluminum risedronate adjuvants with different ratios of zinc, aluminum and risedronate were all milky white suspensions with varying degrees of turbidity; under the electron microscope, most of them were of irregular sheet-like structure, and several of them were of filamentous structure.

### Example 3: Determination of antigenic activity of recombinant SARS-CoV-2 S protein preparation containing FH002C (based on ACE2 receptor binding)

The FH002C (prepared as in Preparation Example 1) and recombinant SARS-CoV-2 S protein were mixed at a volume ratio of 1:1 to form a vaccine preparation, and placed at 4°C for 1 week (complete adsorption) so as to test its antigenicity. The recombinant SARS-CoV-2 S protein stock solution without being adsorbed was used as a control to perform the comparison of antigenicity after the adjuvant was adsorbed and dissociated.

The antigenicity of protein was detected by the in vitro relative potency test based on ACE2 receptor binding:
(1) Antibody coating solution: 1X CB 9.6 buffer (15 mM Na₂CO₃; 35 mM NaHCO₃).
(2) Washing solution: PBST, ELISA kit from INNOVAX Company.
(3) Blocking solution: blocking solution-1, ELISA kit from INNOVAX Company.
(4) Color-developing solution A: ELISA kit from INNOVAX Company.
(5) Color-developing solution B: ELISA kit from INNOVAX Company.
(6) stopping solution: ELISA kit from INNOVAX Company.

### Experiment procedure:

(1) Coating plate: SARS-CoV-2 S protein antibody 45C3 (self-made in the laboratory) was diluted to 1 µg/mL with 1X CB9.6 coating buffer, added at 100 µL/well to a polystyrene 96-well plate, and coating was performed overnight at 4°C.
(2) Blocking: The coating solution in the wells were discarded, the plate was washed once with PBST washing solution, spin-dried, added with blocking solution at 200 µL/well, and blocking was performed at room temperature for 4 h.
(3) Preparation of sample: The recombinant S protein antigen stock solution (before adsorption), and the antigen formed by the dissociation of recombinant S protein vaccine preparation (the concentration of recombinant S protein antigen in the preparation was 100 µg/mL, and the adjuvant was FH002C). The antigen concentration in the first well was 4 µg/mL. The blocking solution in the well was discarded, the plate was washed once with PBST, spin-dried, the sample to be tested was added at 200 µL/well into the first well, 100 µL of the sample diluent solution was added into each subsequent well, 2-fold serial dilution was carried out, and incubation was performed at 25°C for 1 h.
(4) Adding ACE2-hFc (purchased from SinoBiological, Cat. No.:10108-H02H): The liquid in the well was discarded, the plate was washed 5 times with PBST, spin-dried, added with ACE2-hFc, 1 µg/mL, 100 µL/well, and incubation reaction was performed at 25°C for 1 h.
(5) Adding enzyme-labeled antibody (MAH-HRP) (purchased from SouthernBiotech, Cat. No.: 9042-05): The liquid in the well was discarded, the plate was washed 5 times with PBST, spin-dried, added with enzyme-labeled antibody (MAH-HRP, V:V=1:5000), 100 µL/well, and incubation reaction was performed at 25°C for 1 h.
(5) Color development: The enzyme-labeled antibody in the well was discarded, the plate was washed 5 times with PBST, spin-dried, added with 100 µL/well of the mixed color developing solutions A and B in equal volume, and incubation reaction was performed at 25°C for 10 min.
(6) Stopping: 50 µL/well of 2 M sulfuric acid stopping solution was added to stop the reaction.
(7) Plate reading: By setting the detection dual wavelengths at 450 nm and 630 nm on the microplate reader, and the OD value of each reaction well was measured.

The experimental results were shown in Fig. 5. The antigenicity detection results based on ACE2 receptor binding showed that after the FH002C combined with the recombinant SARS-CoV-2 S protein vaccine preparation was dissociated with the dissociation solution, its antigenicity was comparable to the antigenicity of the recombinant S protein stock solution (rEC₅₀ = EC₅₀, stock solution/EC₅₀, after dissociation = 0.9), indicating that the recombinant SARS-CoV-2 S protein vaccine preparation containing FH002C had good stability.

### Example 4: Study on dose-effect relationship of recombinant SARS-CoV-2 S protein containing zinc aluminum risedronate adjuvant

The FH002C adjuvant prepared according to Preparation Example 1 and the Al001 adjuvant prepared according to Preparation Example 3 were used as adjuvants, and mixed with SARS-CoV-2 recombinant recombinant S protein at a volume ratio of 1:1 to form vaccines, respectively, which were then intramuscularly injected into mice, and the specific antibody binding titer and neutralization titer in serum were detected. The specific method was as follows:
Experimental animals: Balb/C mice (purchased from Shanghai Slack Experimental Animal Co., Ltd., Cat. No.: 2017000502480), 6-8 weeks, 5 mice/group, female.
Experimental grouping: (1) low-dose recombinant S protein aqueous solution group (1 µg); (2) high-dose recombinant S protein aqueous solution group (10 µg); (3) low-dose recombinant S protein + Al001 group (1 µg); (4) high-dose recombinant S protein + Al001 group (10 µg); (5) low-dose recombinant S protein + FH002C group (1 µg); (6) high-dose recombinant S protein + FH002C group (10 µg).
Immunization protocol: 1 µg or 10 µg of recombinant S protein per animal, i.e., a vaccine was formed by mixing the adjuvant with recombinant S protein at a volume ratio of 1:1, and then intramuscularly injected into mice, 100 µL per mouse, 50 µL at each hind leg of mouse. Immunization was carried out at the 0^{th} and 3^{rd} weeks, that was, 3 weeks after the first immunization of animals according to the immunization group, blood sample was collected from orbit, and the specific antibody titer in serum was determined. Immunization was boosted at the 3^{rd} week, then blood sample was collected from orbit every week, and the specific antibody binding titer in serum was determined by ELISA method.

Detection of antibody binding titer by enzyme-linked immunosorbent assay (ELISA):
(1) Antibody coating solution: 1X CB 9.6 buffer (15 mM Na₂CO₃; 35 mM NaHCO₃).
(2) Washing solution: PBST, ELISA kit from INNOVAX Company.
(3) Blocking solution: blocking solution-1, ELISA kit from INNOVAX Company.
(4) color-developing solution A: ELISA kit from INNOVAX Company.
(5) color-developing solution B: ELISA kit from INNOVAX Company.
(6) Stopping solution: ELISA kit from INNOVAX Company.

### Experiment procedure:

(1) Coating plate: The antigen recombinant S protein was diluted to a certain concentration with CB9.6 coating buffer, and added at 100 µL/well to a polystyrene 96-well plate, and coating was performed overnight at 4°C.
(2) Blocking: The coating solution in the well was discarded, the plate was washed once with PBST washing solution, spin-dried, added with 200 µL/well of blocking solution, and blocking was performed at room temperature for 4 h.
(3) Adding serum with certain dilution: The blocking solution in the well was discarded, the plate was washed once with PBST, spin-dried, 200 µL/well of the serum to be tested was added into the first well, and 100 µL of the sample diluent was added into each subsequent well, 2-fold serial dilution was carried out, incubation reaction was performed at 25°C for 1 h.
(4) Add enzyme-labeled antibody (GAM-HRP) (purchased from Bio-Rad Laboratories Inc., Cat. No.: 1706516): The serum dilution in the well was discarded, the plate was washed 5 times with PBST, spin-dried, added with enzyme-labeled antibody (GAM-HRP, V:V=1:5000), 100 µL/well, and incubation reaction was performed at 25°C for 1 h.
(5) Color development: The enzyme-labeled antibody in the well was discarded, the plate was washed 5 times with PBST, spin-dried, added with 100 µL/well of the mixed color-developing solutions A and B in equal volume, and reaction was performed at 25°C for 10 min.
(6) Stopping: 50 µL/well of 2 M sulfuric acid stopping solution was added to stop the reaction.
(7) Plate reading: By setting the detection dual wavelengths at 450 nm and 630 nm on the microplate reader, the OD value of each reaction well was measured.

The detection method for the specific antibody neutralization titer in serum was referred to H. L. Xiong et al., Robust neutralization assay based on SARS-CoV-2 S-protein-bearing vesicular stomatitis virus (VSV) pseudovirus and ACE2-overexpressing BHK21 cells. Emerg Microbes Infect, 1-38 (2020).

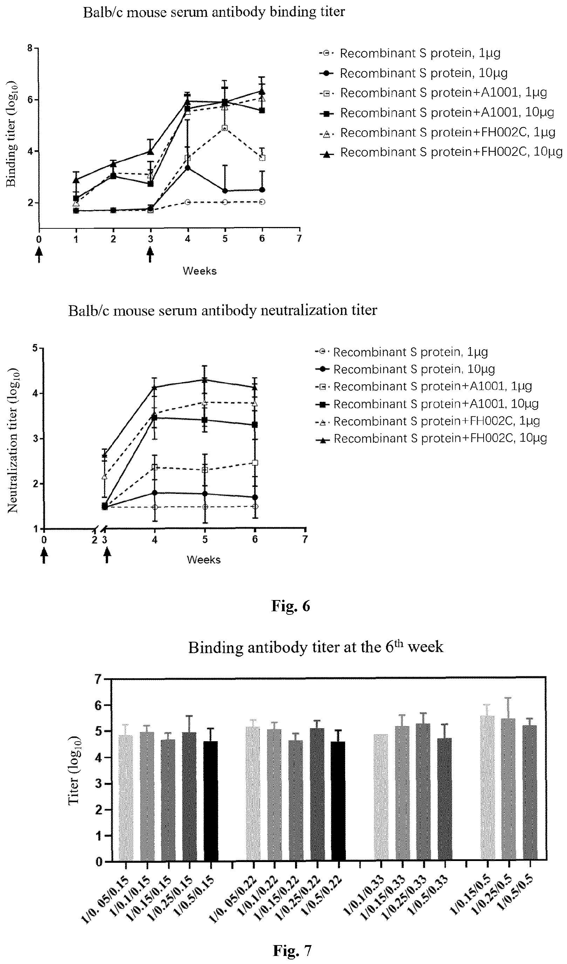

The experimental results were shown in Fig. 6:
Three weeks after the mice were immunized with one shot, under the same immunization dose, the antibody titer of the mice in the FH002C adjuvant group was higher than those in the aqueous solution group and the Al001 adjuvant group, and was higher by 1-1.5 magnitude orders than that in the aluminum adjuvant group, and had the characteristics of taking effect quickly. After two shots of immunization, the humoral immunity-enhancing advantage of the FH002C adjuvant was still obvious. At the 4^{th} week, in the low-dose groups, the antibody titer of the FH002C adjuvant group was higher by 1.5 magnitude orders than that of the aluminum adjuvant group, and higher by 3 magnitude orders than that of the aqueous solution group. In the high-dose groups, the antibody levels of the FH002C adjuvant group and the Al001 group were comparable, and both were higher by 2 magnitude orders than that of the aqueous solution group. At the 5^{th} and 6^{th} weeks, in the high and low dose groups, the antibody titer of the FH002C adjuvant group was still significantly higher than those of the Al001 group (higher by 0.5-1 order of magnitude) and the aqueous solution group (higher by 2.5-3 orders of magnitude).

After one shot, only the mice in the FH002C adjuvant group produced neutralizing antibodies in the 3^{rd} week, while the mice in the aqueous solution group and the Al001 adjuvant group did not produce neutralizing antibodies. After the second shot of immunization, in the 4th, 5th and 6th weeks, whether in the high-dose groups or the low-dose groups, the neutralization titers of the mice in the FH002C adjuvant group were higher than those in the Al001 adjuvant group (higher by 0.5-1.5 orders of magnitude) and the aqueous solution group (higher by 1.5-2.5 orders of magnitude).

### Example 5: Specific antibody binding titer induced by FH002C adjuvant with different ratios of zinc, aluminum, and risedronate combined with recombinant SARS-CoV-2 S protein

The prepared FH002C (see Preparation Example 2 for the preparation method) was used as an adjuvant and mixed with the recombinant SARS-CoV-2 S protein at a volume ratio of 1:1 to form a vaccine, which was then intramuscularly injected into Balb/C mice to measure the titer of the specific antibody as produced. The specific method was as follows:
Experimental animals: Balb/C mice, 6-7 weeks old, female, 5 in each group
Immunization protocol: 1 µg of antigen per mouse, i.e., 100 µL per mouse (half for left leg and half for right leg). Two shots of immunization were carried out in the 0^{th} and 3^{rd} weeks, and from the 0^{th} to 6^{th} weeks, orbital blood was collected weekly to perform the detection of antibody titer in serum.

Enzyme-linked immunosorbent assay (ELISA) was used to detect antibody binding titer, which was specially described as follows:
The reagents used in ELISA were referred to Example 4.

### Experiment procedure:

(1) Coating and blocking: The recombinant S protein was diluted to 1 µg/mL with CB9.6 and added to a 96-well microtiter plate at 100 µL/well, placed and incubated at 37°C for 2 hours, and then the plate was washed once with PBST buffer (i.e., PBS containing 0.05% Tween-20), 200 µL of PBS containing 20% NBS was added into each well, and then blocking was performed by incubating at 37°C for 2 h. After removing excess liquid, it was spin-dried for later use.
(2) Dilution of serum: 100 µL of 300-fold diluted serum was added to the first well, then 3-fold diluted at 10 gradients, and incubated at 37°C for 1 h.
(3) Adding enzyme-labeled antibody: After the plate was washed 5 times with PBST buffer, 100 µL of goat-anti-mouse-horseradish peroxidase enzyme-labeled antibody GAM-HRP (self-made in the laboratory) diluted by certain folds was added, and incubated at 37°C for 0.5 h.
(4) Color development, stopping, and reading.

Experimental results: As shown in Fig. 7, the FH002C adjuvants with different ratios of zinc, aluminum, and risedronate combined with the recombinant SARS-CoV-2 S protein all could induce specific binding antibodies.

### Example 6: Specific antibody binding titer induced by zinc aluminum risedronate adjuvant combined with recombinant SARS-CoV-2 S protein

The prepared Al001 (Preparation Example 3) and FH002C (Preparation Example 1) were used as adjuvants and mixed with the recombinant SARS-CoV-2 S protein at a volume ratio of 1:1 to form vaccines, and then intramuscularly injected into Syrian golden hamsters (also referred to herein as "Syrian hamsters") and cynomolgus monkeys to determine the specific antibody titers as produced. The specific method was as follows:
Experimental animals: Syrian golden hamsters (purchased from Shanghai Slack Experimental Animal Co., Ltd.), 6-7 weeks, 22/group for FH002C group, 16/group for Al001 group, half male and half male. Adult cynomolgus monkeys (purchased from Guangxi Xiongsen Primate Experimental Animal Breeding and Development Co., Ltd.), 2/group, half male and half male.
Experimental grouping: (1) recombinant S protein + Al001; (2) recombinant S protein + FH002C
Immunization protocol for Syrian golden hamsters: 10 µg of antigen per animal, i.e., vaccines were formed by separately mixing the adjuvants with recombinant SARS-CoV-2 S protein at a volume ratio of 1:1, and then intramuscularly injected into Syrian golden hamsters, 200 µL per animal. Three immunization shots were carried out at the 0^{th}, 2^{nd} and 6^{th} weeks, and from the 0^{th} to 7^{th} weeks, orbital blood was collected weekly for the detection of antibody titer in serum.
Immunization protocol for cynomolgus monkeys: 20 µg of antigen per monkey, i.e., vaccines were formed by separately mixing the adjuvants with recombinant SARS-CoV-2 S protein at a volume ratio of 1:1, and then intramuscularly injected into cynomolgus monkeys, 500 µL per animal. Two immunization shots were carried out at the 0^{th} and 2^{nd} weeks, and from the 0^{th} to 6^{th} weeks, blood was collected weekly for the detection of antibody binding titer.

Enzyme-linked immunosorbent assay (ELISA) was used to detect antibody binding titers, which was specifically described as follows:
The reagents used in ELISA were referred to Example 4.

### Experiment procedure:

(1) Coating and blocking: The recombinant S protein was diluted to 2 µg/mL with CB9.6 and added to a 96-well microtiter plate at 100 µL/well, placed and incubated at 37°C for 2 hours, and then the plate was washed once with PBST buffer (i.e., PBS containing 0.05% Tween-20), 200 µL of PBS containing 20% NBS was added to each well, and then incubated at 37°C for 2 h for blocking. After removing excess liquid, it was spin-dried for later use.
(2) Dilution of serum: 100 µL of serum 50- or 100-fold diluted was added to each well, and incubated at 37°C for 1 h.
(3) Adding enzyme-labeled antibody: After the plate was washed 5 times with PBST buffer, 100 µL of HRP-coupled goat-anti-human IgG (H + L) (BEYOTIME, A0201) or goat-anti-Syrian golden hamster IgG H&L (Abeam, ab6892), which was diluted by a certain fold, was added, and incubated at 37°C for 0.5 h.
(4) Color development, stopping, and reading.

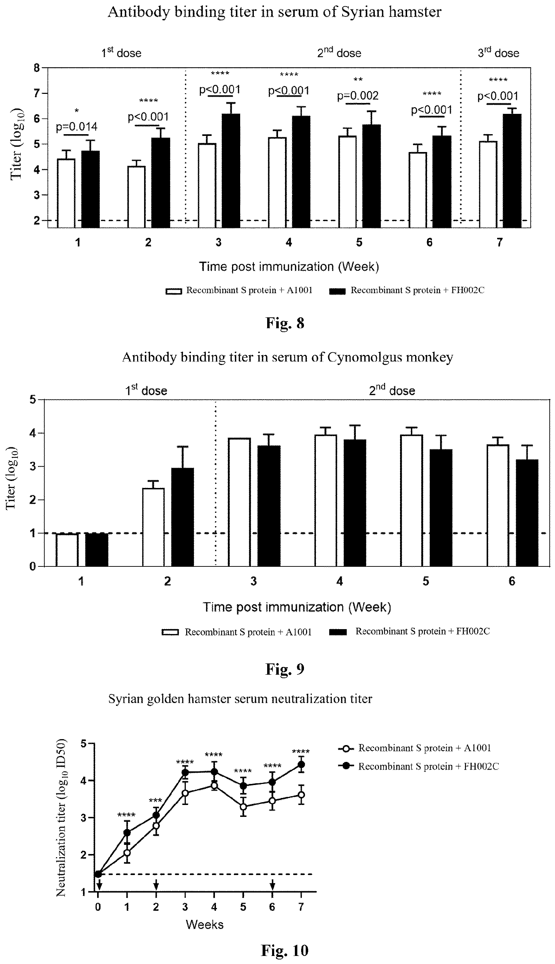

Experimental results: As shown in Fig. 8, two weeks after the Syrian golden hamsters were immunized with one shot, the antibody titer of the FH002C group was higher than that of the Al001 group, that was, 15 times that of the aluminum adjuvant group, and the FH002C had the characteristics of taking effect rapidly. After two shots of immunization, its advantage of enhancing humoral immunity was still obvious. At the 4^{th} week, the antibody titer of the FH002C group was 8 times that of the aluminum adjuvant group. After three injections, the antibody titer of the FH002C group was 11 times that of the control group at the 7^{th} week.

Two weeks after immunization of cynomolgus monkeys, the antibody titer of the FH002C group was higher than that of the Al001 group, that was, 6 times that of the aluminum adjuvant group, and the FH002C had the characteristics of taking effect rapidly. After two shots of immunization, at the 4^{th} week, the antibody titer of the FH002C group was comparable to that of the aluminum adjuvant group (Fig. 9).

### Example 7: Neutralizing antibody titer induced by zinc aluminum risedronate adjuvant combined with recombinant SARS-CoV-2 S protein

The experimental procedure described in Example 6 was adopted, the adjuvants Al001 (Preparation Example 3) and FH002C (Preparation Example 1) were combined with the recombinant SARS-CoV-2 S protein, respectively, and used to immunize Syrian golden hamsters and cynomolgus monkeys by intramuscular injection, and neutralizing antibody titers in serum were detected. The immunization procedures for Syrian golden hamsters and cynomolgus monkeys were the same as that in Example 6.

Antibody neutralization titers were detected by Pseudovirus neutralization (H. L. Xiong et al., Robust neutralization assay based on SARS-CoV-2 S-protein-bearing vesicular stomatitis virus (VSV) pseudovirus and ACE2-overexpressing BHK21 cells. Emerg Microbes Infect, 1-38 (2020)), which was specifically described as follows:
Vesicular stomatitis virus (VSV) pseudovirus (rVSV-SARS-CoV-2) bearing SARS-CoV-2 S protein (Wuhan-Hu-1 strain, gene bank:QHD43416.1) was packaged by recombinant plasmid pCAG-nCoVSdel18 and VSVdG-EGFP-G virus (Addgene, 31842). The hACE2-expressing BHK21 (BHK21-hACE2) cells were spread into 96-well plates 24 h in advance, then the serum was diluted by 50-fold with high-glucose DMEM medium (Sigma-Aldrich, D6429) containing 10% FBS (GIBCO, 10099141) and penicillin-streptomycin (GIBCO, 15140122), and then 3-fold serial dilution was performed. The diluted rVSV-SARS-CoV-2 virus (MOI = 0.05) was mixed with the diluted serum, and incubated at 37°C for 1 h. Immediately afterwards, the mixture was transferred to a plate coated with BHK21-hACE2 cells, and then incubated in an incubator containing 5% CO₂ at 37°C for 12 h. Fluorescence image data were captured by Opera Phenix or Operetta CLS high content analysis system (PerkinElmer), and the count of GFP-positive cells per well was analyzed by Columbus system (PerkinElmer). Each plate contained 8 serum-free wells as virus controls. The serum neutralization titer was defined as the serum dilution fold (ID₅₀) corresponding to that the number of GFP-positive cells was reduced by 50% compared with the positive wells.

Experimental results: As shown in Fig. 10, two weeks after the Syrian golden hamster was immunized by one shot, the antibody titer of the FH002C group was higher than that of the Al001 group, that was, 1.8 times that of the aluminum adjuvant group, and the FH002C had the characteristics of taking effect rapidly. After two shots of immunization, its advantage of enhancing humoral immunity was still obvious. At the 4^{th} week, the antibody titer of the FH002C group was 2.6 times that of the aluminum adjuvant group. After three shots, the antibody titer of the FH002C group was 6.5 times that of the control group at the 7^{th} week.

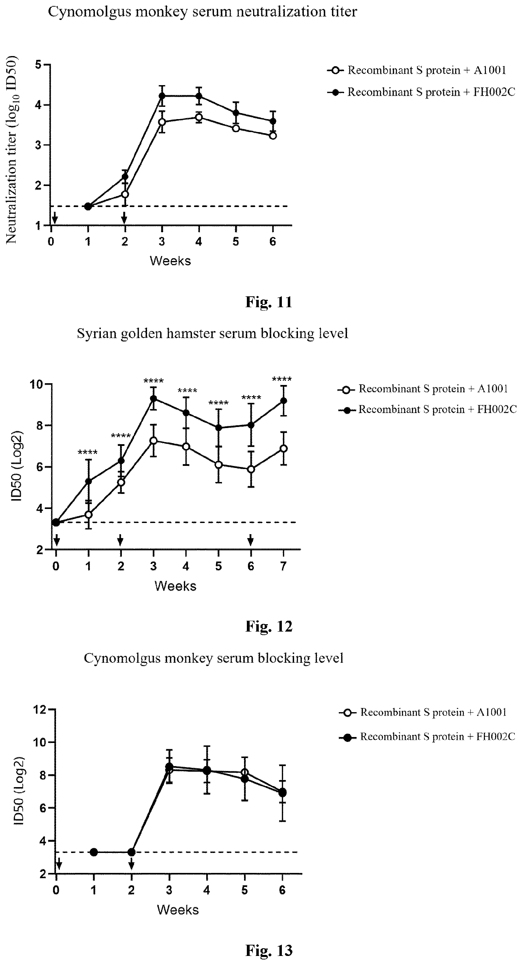

Two weeks after immunization of cynomolgus monkeys, the antibody titer of the FH002C group was higher than that of the Al001 group, that was, 2.6 times that of the aluminum adjuvant group. After two shots of immunization, the antibody titer of the FH002C group was 3.5 times that of the aluminum adjuvant group at the 4^{th} week (Fig. 11).

### Example 8: Cell-based Spike blocking experiment of immune serum induced by zinc aluminum risedronate adjuvant combined with recombinant SARS-CoV-2 S protein

The immunization procedure described in Example 6 was adopted, the adjuvants Al001 (Preparation Example 3) and FH002C (Preparation Example 1) were combined with the recombinant SARS-CoV-2 S protein to immunize Syrian golden hamsters and cynomolgus monkeys by intramuscular injection, and cell-based Spike serum blocking experiment (Y. Zhang et al., Virus-free and live-cell visualizing SARS-CoV-2 cell entry for studies of neutralizing antibodies and compound inhibitors. bioRxiv, (2020)) was performed as follows:
293T Cells expressing hACE2-mRuby3 (293T-ACE2iRb3) were spread on 96-well plates pretreated with poly-D-lysine at 2×10⁴/well 24 h in advance. The sera of Syrian golden hamster and cynomolgus monkey were diluted by 2-fold serial dilution with DMEM medium containing 10% FBS. 11 µL of probe of SARS-CoV-2 spike trimer fused with Gamillus (STG) was mixed with 44 µL of the diluted serum so that the final probe concentration was 2.5 nM. Half of the culture medium was removed from the wells plated with 293T-ACE2iRb3 cells, 50 µL of the mixture was pipetted and added into the cells, and incubated at 37°C, 5% CO₂ for 1 h. Subsequently, cell images were captured in confocal mode by Opera Phenix high-content analysis system.

Data analysis: Columbus System was used for quantitative analysis of the image data. First, according to the principle that 293T-ACE2iRb3 cells all expressed H2B-fused iRFP670 localized in the nucleus, all inoculated cells were found through the near-infrared channel (Ex:640/Em:670). According to the principle that hACE2 fused with mRuby3 would be located on the membrane, the cell boundary was defined by the red channel (Ex:561/Em:590). Then by calculating the MFI of the STG probe channel (Ex:488/Em:525) in the cytoplasmic area (cMFI, outer boundary: 20%, inner boundary: 45%), the inhibition ratio could be obtained: [(cMFIpc-cMFItst)/(cMFIpc-cMFIblk)] × 100%. Wherein, cMFItst referred to the cMFI value of test well, cMFIpc and cMFIblk referred to the cFMI values of probe-only (positive control) well and cell-only well, respectively. The serum blocking level was expressed by ID₅₀.

Experimental results: As shown in Fig. 12, the serum blocking rate of the Syrian golden hamster FH002C group was higher than that of the Al001 group; as shown in Fig. 13, in cynomolgus monkeys, the serum blocking rates of the two adjuvant groups were comparable.

### Example 9: Effect of zinc aluminum risedronate adjuvant combined with recombinant SARS-CoV-2 S protein on antibody affinity

The experimental procedure described in Example 6 was adopted, the adjuvants Al001 (Preparation Example 3) and FH002C (Preparation Example 1) were mixed with the recombinant SARS-CoV-2 S protein to immunize mice and Syrian golden hamsters by intramuscular injection, and serum antibody affinities were detected. The immunization procedure of Syrian golden hamsters was the same as in Example 6, but each of the FH002C and Al001 groups had 16 animals, half male and half male. The immunization procedure of mice was as follows:
Experimental animals: Balb/C mice (purchased from Shanghai Slack Experimental Animal Co., Ltd.), 6-8 weeks, 4 or 5 mice/group, female.
Experimental grouping: (1) recombinant S protein + Al001; (2) recombinant S protein + FH002C.
Immunization protocol of mice: 1 µg of antigen per animal, i.e., vaccines were formed by separately mixing the adjuvants with recombinant SARS-CoV-2 S protein at a volume ratio of 1:1, and then intramuscularly injected into Balb/C mice, 150 µL per animal. Three shots of immunization were carried out at the 0^{th}, 2^{nd}, and 4^{th} weeks, and from the 0^{th} to 6^{th} weeks, orbital blood was collected weekly for the detection of antibody affinity in serum.

The antibody affinity was detected by enzyme-linked immunosorbent assay (ELISA), which was described in detail as follows:
The reagents used in ELISA were referred to Example 4.

### Experiment procedure:

Coating and blocking: The purified recombinant S protein was diluted to 2 µg/mL with CB9.6 and then used for coating, added at 100 µL/well to ELISA plates, placed and incubated at 37°C for 2 h, and then the plate was washed once with PBST buffer (i.e., PBS containing 0.05% Twenn-20), 200 µL of PBS containing 20% NBS was added to each well, and the placed and incubated at 37°C for 2 h for blocking. After removing excess liquid, it was spin-dried.

Detection of serum antibody affinity: 100 µL of serum was added to each well, double well repeats were performed for each sample, and incubation was performed at 37°C for 1 h. After the plate was washed once with PBST buffer, 100 µL of PBS was added to one well of the double well repeats, and 100 µL of 4M urea (prepared in PBS) was added to the other well, and the plate was allowed to stand at 37°C for 20 min, and then washed 4 times with PBST buffer. Then HRP-conjugated goat-anti-mouse IgG (H + L) (Proteintech, SA00001-1) or goat-anti-Syrian hamster IgG H&L (Abeam, ab6892), that was diluted by a certain fold, was added and incubated at 37°C for 0.5 h. The plate was washed 5 times with washing buffer. 100 µL of color-developing substrate mix was added to each well, and the absorbance at 450 nm was read by PHOMO Microplate reader. The antibody avidity could be expressed by antibody ratio, that was, the ratio of the EC₅₀ of the urea-treated group to the EC₅₀ of the untreated group.

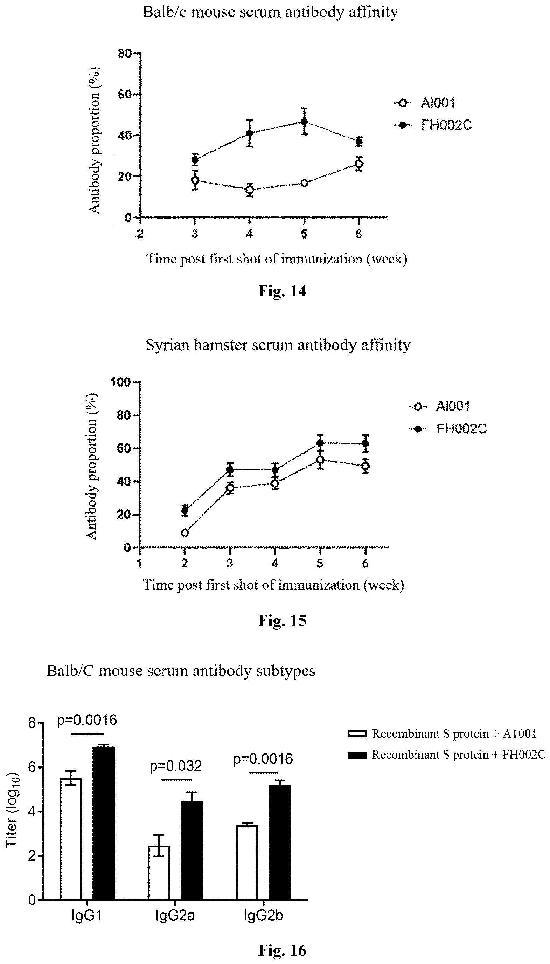

Experimental results: As shown in Figs. 14 and 15, regardless of the mouse model or the Syrian golden hamster model, the FH002C group could produce a higher proportion of high-affinity antibodies than the Al001 group.

### Example 10: Production of specific antibody subtypes by immunizing mice with zinc aluminum risedronate adjuvant combined with recombinant SARS-CoV-2 S protein

The prepared Al001 (preparation example 3) and FH002C (preparation example 1) were used as adjuvants and mixed with the recombinant SARS-CoV-2 S protein at a volume ratio of 1:1 to form vaccines, which were then intramuscularly injected into mice. The immunization procedure was as in Example 9.

Enzyme-linked immunosorbent assay (ELISA) was used to detect the level of antibody subtypes, which was described in detail as follows:
The reagents used in ELISA were referred to Example 4.

### Experiment procedure:

Coating and blocking: The purified recombinant S protein was diluted to 2 µg/mL with carbonate buffer, added at 100 µL/well to ELISA plates, and incubated at 37°C for 2 h, and then the plate was washed once with PBST buffer (i.e., PBS containing 0.05% Tween-20), and 200 µL of PBS containing 20% NBS was added to each well, and incubated at 37°C for 2 h for blocking. After removing excess liquid, it was spin-dried.

Detection of serum antibody subtype level: 100 µL of serum was added to each well, incubated at 37°C for 1 h. After the plate was washed 5 times with PBST buffer, 100 µL of HRP-coupled goat-anti-mouse IgG1 (AbD Serotec, STAR132P) or goat-anti-mouse IgG2a (AbD Serotec, STAR133P) or goat-anti-mouse IgG2b (AbD Serotec, STAR134P) was added, incubated at 37°C for 0.5 h. The plate was washed 5 times with washing buffer. 100 µL of 3-fold diluted color-developing substrate mix was added to each well and the absorbance at 450 nm was read by PHOMO Microplate reader. Each plate contained 5 wells of negative serum as negative control.

Experimental results: As shown in Fig. 16, compared with the aluminum adjuvant group, the FH002C adjuvant group could induce higher levels of IgG2a and IgG2b subtype antibodies, and the ratios of IgG1 to IgG2a and IgG2b were lower than those of the aluminum adjuvant group, indicating that it had a certain stimulating effect on the Th1 immune pathway.

### Example 11: Effect of zinc aluminum risedronate adjuvant combined with recombinant SARS-CoV-2 S protein on T cell response

The prepared Al001 (Preparation Example 3) and FH002C (Preparation Example 1) were used as adjuvants and mixed with the recombinant SARS-CoV-2 S protein at a volume ratio of 1:1 to form vaccines, which were then intramuscularly injected into mice to measure the induced T cells response. The specific method was as follows:
Experimental animals: C57BL/6, 6-8 weeks, 8 animals/group, female.
Experimental grouping: (1) blank group; (2) recombinant S protein + Al001; (3) recombinant S protein + FH002C.
Immunization protocol for C57BL/6 mice: 10 µg of antigen per animal, i.e., vaccines were formed by mixing the adjuvants with recombinant SARS-CoV-2 S protein at a volume ratio of 1:1, and then injected intramuscularly, 150 µL per animal. Two shots of immunization were carried out at the 0^{th} and 3^{rd} weeks, and the spleen and lymph nodes were separated after sacrifice at the 4^{th} week for the assay of T cell immune response.

Enzyme-linked immunospot assay (ELISPOT) was used to detect T cell response.

Spleen and lymph nodes of mice were taken, prepared into a single cell suspension, and spread on mouse IFN-γ-coated ELISPOT plates (DAKEWEI, 2210005) at 10⁶ (spleen) cells per well or 4×10⁵ (lymph nodes) cells per well. Then, they were stimulated and cultured with PBS or a 15-unit SARS-CoV-2 S peptide library with 11 amino acid overlaps (Genscript, RP30020) for 20 h. Subsequently, the detection was performed according to the kit instructions. Image capture and spot counting were performed using CTL-ImmunoSpot^{®} S5 (Cellular Technology Limited). The number of IFN-γ-secreting cells was calculated by subtracting PBS-stimulated wells from spike peptide library-stimulated wells.

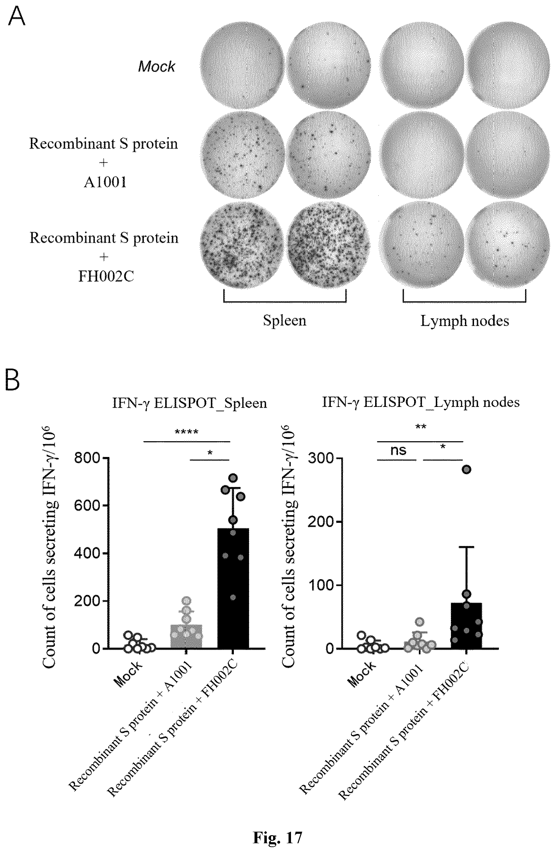

Experimental results: As shown in Fig. 17, the number of cells secreting IFN-γ in the FH002C and Al001 groups increased by 28.9 and 5.8 times in the spleens, respectively, and increased by 14.0 and 2.3 times in the lymph nodes. Compared with the Al001 group, the FH002C group showed a higher level of induced T cell responses.

### Example 12: Specific antibody binding titers induced by aluminum adjuvant (A1001) and FH002C adjuvant combined with varicella zoster virus gE protein (VZV gE)

The prepared Al001 (Preparation Example 3) and FH002C (Preparation Example 1) were used as adjuvants and mixed with varicella zoster virus gE protein (VZV gE, SEQ ID NO:2) at a volume ratio of 1:1 to form vaccines, which were then injected intramuscularly into Balb/C mice, and the specific antibody titers as produced were detected. Wherein, the gE protein was obtained by expression using an *Escherichia coli* expression system (purchased from Shanghai Weidi Biotechnology Co., Ltd., EC1060).

The specific method was as follows:
Experimental animals: Balb/C mice, 6-7 weeks old, 5 in each group, female.
Experimental grouping: (1) VZV gE+Al001; (2) VZV gE+FH002C.
Immunization protocol for Balb/C mice: 5 µg of antigen per mouse, i.e., vaccines were formed by mixing the adjuvants with varicella zoster virus gE protein (VZV gE) at a volume ratio of 1:1, and then intramuscularly inj ected into Balb/C mice, 100 µL per animal. Two shots of immunization were carried out at the 0^{th}, 2^{nd}, and 4^{th} weeks, and from the 0^{th} to 4^{th} weeks, orbital blood was collected weekly for the detection of antibody titer in serum.

Enzyme-linked immunosorbent assay (ELISA) was used to detect antibody binding titers, which was described in detail as follows:
The reagents used in ELISA were referred to Example 4.

### Experiment procedure:

(1) Coating and blocking: VZV gE was diluted to 1 µg/mL with PB7.4 + NaCl and added to 96-well microplate at 100 µL/well, incubated overnight at 4°C, and then the plate was washed once with PBST buffer (i.e., PBS containing 0.05% Tween-20), 200 µL of PBS containing 20% NBS was added to each well, and then incubated at 37°C for 2 h for blocking. After removing excess liquid, it was spin-dried for later use.
(2) Dilution of serum: 100 µL of serum diluted by 50 or 100 fold was added to each well, and incubated at 25°C for 1 h.
(3) Adding enzyme-labeled antibody: After the plate was washed 5 times with PBST buffer, 100 µL of enzyme-labeled antibody (GAM-HRP) (purchased from Bio-Rad Laboratories Inc., Cat. No.: 1706516) was added, and incubated at 25°C for 1 h.
(4) Color development, stopping, and reading.

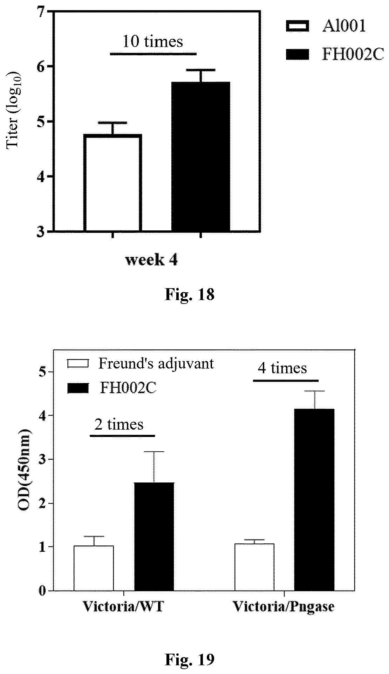

Experimental results: As shown in Fig. 18, after two shots of immunization, the binding titer of the FH002C adjuvant group was significantly higher by about 10 times than that of the Al001 group.

### Example 13: Specific antibody binding titer induced by Freund's adjuvant and FH002C adjuvant combined with influenza virus HA protein

Commercially available Freund's adjuvant (purchased from SIGMA-ALDRICH, Cat. No.: F5881), the FH002C adjuvant (preparation example 1) were mixed with influenza virus HA protein (SEQ ID NO:3) treated with or without deglycosylation enzyme at a volume ratio of 1:1 to form vaccines, which were then subcutaneously injected into Balb/C mice to measure the specific antibody titer produced. Wherein, the influenza virus HA protein was obtained by expression using a baculovirus expression system (purchased from Invitrogen, USA, 10359016). The deglycosylation enzyme was purchased from NEB, Cat. No.: P0705S; the deglycosylation enzyme treatment process was carried out according to the instructions of the deglycosylation enzyme.

The specific method was as follows:
Experimental animals: Balb/C mice (purchased from Shanghai Slack Experimental Animal Co., Ltd.), 6-7 weeks, 10 mice/group in the FH002C group, 10 mice/group in the Freund's adjuvant group.
Experimental grouping: (1) HA + Freund's adjuvant; (2) HA + FH002C.
Immunization protocol: (1) 30 µg of antigen per animal, i.e., a vaccine was formed by mixing Freund's adjuvant with influenza virus HA protein at a volume ratio of 1:1, and then subcutaneously injected into Balb/C mice, 300 µL per animal; (2) 30 µg of antigen per animal, i.e., a vaccine was formed by mixing FH002C with influenza virus HA protein at a volume ratio of 1:1, and then intramuscularly injected into Balb/C mice, 200 µL per animal. 14 days after immunization, orbital blood was collected for the detection of antibody titer in serum.

Enzyme-linked immunosorbent assay (ELISA) was used to detect antibody binding titer, which was described in detail as follows:
The reagents used in ELISA were referred to Example 4.
(1) Coating and blocking: The recombinant HA protein or ultracentrifuged influenza virus was diluted to 2 µg/mL with CB9.6, or added at 100 µL/well to a 96-well microtiter plate, placed and incubated at 37°C for 2 hours, and then the plate was washed once with PBST buffer (i.e., PBS containing 0.05% Tween-20), and 200 µL of commercial blocking solution was added to each well, and then incubated at 37°C for 2 h for blocking. After removing excess liquid, it was spin-dried for later use.
(2) Detection of serum: 100 µL of serum diluted by different fold was added to each well, and incubated at 37°C for 1 h.
(3) Adding enzyme-labeled antibody: After the plate was washed 5 times with PBST buffer, 100 µL of enzyme-labeled goat-anti-mouse antibody GAM-HRP (self-made in the laboratory) diluted by 5000-fold was added, and incubated at 37°C for 0.5 h.
(4) Color development, stopping, and reading.

Experimental results: As shown in Fig. 19, two weeks after Balb/C mice were immunized with one shot, for the influenza virus HA protein treated with and without deglycosylation enzyme, the antibody titers of the FH002C adjuvant group were all higher than those of the Freund's adjuvant group, and the FH002C adjuvant had the characteristics of taking effect rapidly.

### Example 14: Neutralizing antibody titers induced by aluminum adjuvant (A1001) and FH002C adjuvant combined with rotavirus VP4 protein

The prepared Al001 (preparation example 3) and FH002C (preparation example 1) were used as adjuvants and mixed with rotavirus VP4 protein at a volume ratio of 1:1 to form vaccines, which were then intramuscularly injected into Balb/C mice and guinea pigs for determining the neutralizing antibody titers as produced. Wherein, the VP4 protein was expressed by an *Escherichia coli* expression system (purchased from Shanghai Weidi Biotechnology Co., Ltd., EC 1060).

The specific method was as follows:
Experimental animals: Balb/C mice, purchased from Shanghai Slack Experimental Animal Co., Ltd., 6-8 weeks old; guinea pigs, 450-500g, purchased from Shanghai Songlian Experimental Animal Farm.
Mice groups: 5 mice/group in the FH002C group, 5 mice/group in the Al001 group, all animals were female.
Guinea pig groups: 5 guinea pigs/group in the FH002C group, 5 guinea pigs/group in the Al001 group, all animals were female.
Experimental grouping: (1) VP4+Al001; (2) VP4+FH002C.
Immunization protocol: 10 µg of antigen per animal for mouse immunization, 10 µg of antigen per animal for guinea pig immunization, i.e., vaccines were formed by separately mixing the adjuvants with rotavirus VP4 protein at a volume ratio of 1:1, and then intramuscularly injected into mice and guinea pigs, 200 µL per animal for both mice and Guinea pigs. Before the first shot of immunization and 2 weeks after the third shot of immunization, blood was collected and serum was separated for the detection of neutralizing antibody titer.

Enzyme-linked immunospot assay (ELISPOT) was used to detect neutralizing antibody titers, which was described in detail as follows:
(1) Spreading MA104 cells on 96-well cell plate: After the cells were digested with trypsin solution, the cells were suspended by pipetting with a cell culture medium containing 10% FBS, then counting was carried out using a cell counting plate, the culture medium was added for dilution to 25,000 cells/mL, and then it was evenly added to a 96-well plate, 100 µL/well, and incubated at 37°C for 20 h, with a CO₂ concentration of 5%.
(2) Digestion treatment of virus: 4 µL of 2.5 µg/µL trypsin was added to 1 mL of rotavirus solution, mixed well and placed for treatment; after treatment, the virus was diluted with serum-free DMEM culture medium containing 1 µg/mL trypsin to a certain titer.
(3) Inactivation treatment of serum complement: 10 µL of serum sample was taken and placed in a 1.5 mL EP tube, and then heat-treated at 56°C for 30 min. After the treatment, the serum sample was diluted with DMEM containing 1 µg/ml trypsin, and double well repeats were performed for the detection.
(4) Serum-virus reaction: 100 µL of the virus in (2) was mixed with 100 µL of the serum in (3), and neutralization reaction was performed at 37°C for 1 h.
(5) Replacement of cell culture medium: The cell supernatant in (1) was removed, the MA104 cells in (1) were rinsed with serum-free DMEM medium containing 1 µg/mL trypsin for 5 min, and the washing was repeated for three times.
(6) Infection: After the last rinsing, the rinsing medium was removed, the neutralization reaction mixture in (4) was added to the cells, and incubated at 37°C for 14 h with a CO₂ concentration of 5%.
(7) Cell fixation: The cell supernatant in (6) was gently spin-dried to avoid cell damage, then the cells were fixed with PBS solution containing 0.1% glutaraldehyde, 100 µL/well, and the fixation was performed at room temperature for 1 h, in which the fixation should be performed under dark conditions to avoid light, that could prevent the photodecomposition of glutaraldehyde from affecting the fixation effect.
(8) Cell permeabilization: The glutaraldehyde fixation solution in (7) was removed, 100 µL of PBS solution containing 0.3% Triton X-100 was added, and permeabilization treatment was performed at room temperature for 30 min.
(9) Oxidation: The TritonX-100 permeabilization solution in (8) was removed, then 100 µL of 3% H₂O₂ in PBS solution was added, and treatment was performed at room temperature for 15 min.
(10) Washing: The oxidization solution was removed, and the cells were rinsed with PBST washing solution 5 times, 5 min each time.
(11) Enzyme-labeled antibody reaction: The rinse solution was removed by spin-drying, and then the HRP-labeled VP6 antibody (self-made in the laboratory, the monoclonal antibody was an antibody that recognized VP6, and prepared by hybridoma technology, the method was referred to Li, et al. Emerging Microbes Infection. 2020) was diluted with the previously prepared enzyme-labeled antibody reaction solution (enzyme diluent, ED-13), with a dilution ratio of 1:5000, and added to the cells, and reaction was performed at 37°C for 1 h.
(12) Washing: The enzyme-labeled antibody reaction solution was removed, and the cells were rinsed with PBST washing solution 5 times, 5 min each time.
(13) Color development: The rinsing solution was spin-dried, and then the TMB color-developing solution as prepared on site was added, 100 µL/well, and reacted at room temperature for 15 minutes in the dark to avoid the reaction of the color-developing solution with light, which could affect the experimental results.
(14) Plate reading: The color-developing solution was spin-dried, and ELISPOT plate reader was used to read and count the above 96-well plate.

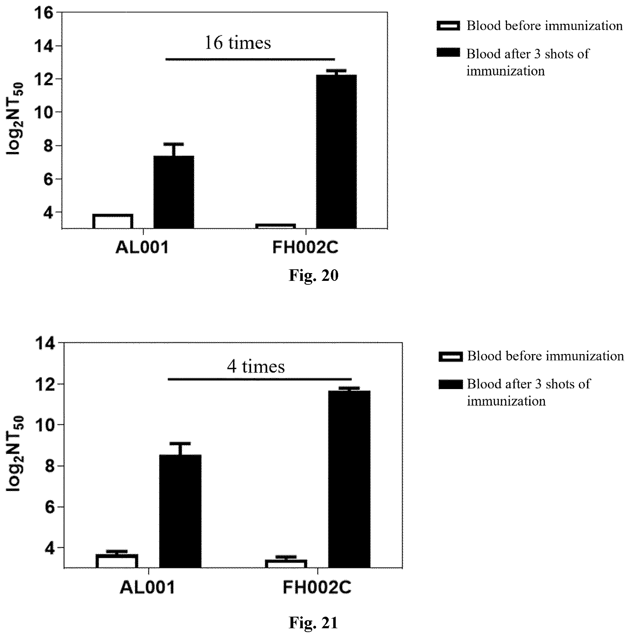

Experimental results: As shown in Figs. 20 and 21, both mice and guinea pigs could produce neutralizing antibodies after three shots of immunization, and the neutralizing antibody titers in the FH002C group were higher than those in the A1001 group. Among them, in the mice, the serum neutralization titer of the FH002C adjuvant group was about 16 times that of the A1001 adjuvant group (Fig. 20); while in the guinea pigs, the serum neutralization titer of the FH002C adjuvant group was about 4 times that of the A1001 group times (Fig. 21).

Although the specific embodiments of the present invention have been described in detail, those skilled in the art will understand that: according to all the teachings that have been published, various modifications and changes can be made to the details, and these changes are all within the protection scope of the present invention. The full scope of the present invention is given by the claims appended hereto and any equivalents thereof.

## Claims

1. An adjuvant comprising zinc aluminum risedronate, wherein the adjuvant has a zinc:risedronate molar concentration ratio in the range of 1:1 to 16:1 (e.g., in the range of 2:1 to 16:1); and, has a zinc:aluminum molar concentration ratio in the range of 1:1 to 50:1 (e.g., in the range of 5:1 to 50:1);
preferably, the adjuvant is in the form of particles; preferably, the particles have a particle size of 0.01-100 µm, such as 0.01-60 µm, 0.01-50 µm, 0.1-60 µm, 0.1-30 µm, 0.4-30 µm, 0.4 -20 µm;
preferably, the adjuvant has a zinc:risedronate molar concentration ratio in the range of 1:1 to 2:1, 2:1 to 4:1, 4:1 to 6:1, 6:1 to 8:1, 8:1 to 10:1, 10:1 to 12:1, 12:1 to 14:1, or 14:1 to 16:1; preferably, the adjuvant has a zinc:risedronate molar concentration ratio of 4:1 or 4.5:1;
preferably, the adjuvant has a zinc:aluminum molar concentration ratio in the range of 1:1 to 2:1, 2:1 to 3:1, 3:1 to 4:1, 4:1 to 5:1, 5:1 to 6:1, 6:1 to 8:1, 8:1 to 10:1, 10:1 to 12:1, 12:1 to 15:1, 15:1 to 20:1, 20:1 to 30:1, 30:1 to 40:1, or 40:1 to 50:1; preferably, the adjuvant has a zinc:aluminum molar concentration ratio of 10:1;
preferably, the adjuvant has a zinc:risedronate molar concentration ratio in the range of 2:1 to 8:1, such as 2:1 to 4:1, 4:1 to 6:1, or 6:1 to 8:1, and a zinc:aluminum molar concentration ratio in the range of 2:1 to 50:1 (e.g., in the range of 5:1 to 20:1), such as 2:1 to 3:1, 3:1 to 4:1, 4:1 to 5:1, 5:1 to6:1,6:1 to8:1,8:1 to 10:1, 10:1 to 12:1, 12:1 to 15:1, or 15:1 to 20:1;
preferably, the adjuvant has a zinc:risedronate molar concentration ratio of 4:1, and a zinc:aluminum molar concentration ratio of 10:1;
preferably, the adjuvant has a zinc:risedronate molar concentration ratio of 4.5:1, and a zinc:aluminum molar concentration ratio of 10:1;
preferably, the adjuvant has a pH of 5.0-8.0, such as 5.0-7.0, 5.0-5.5, 5.5-6.0, 6.0-6.5, 6.5-7.0, 7.0-7.5, or 7.5-8.0;
preferably, the adjuvant has a zero charge point of 3.0-8.0, such as 4.0-8.0, 3.0-4.0, 4.0-5.0, 5.0-6.0, 6.0-7.0, or 7.0-8.0;
preferably, the adjuvant has an adsorption rate of at least 60%, at least 70%, at least 75%, at least 80%, at least 85%, at least 90%, or at least 95% for an immunogen (e.g., protein).

2. A method for preparing the adjuvant according to claim 1, which comprises the following steps:
1) providing a soluble salt solution containing zinc ions and aluminum ions;
2) mixing the soluble salt solution in step (1) with an alkaline risedronate solution to obtain the adjuvant;
preferably, the method further comprises a step of sterilizing the adjuvant obtained in step (2); preferably, the adjuvant is sterilized by a filter sterilization or a high-temperature and highpressure sterilization, for example, the adjuvant is sterilized at 121°C for at least 15 minutes (e.g., at least 30 minutes, for example, 30-60 minutes);
preferably, the soluble salt solution has a zinc:aluminum molar concentration ratio in the range of 1:1 to 50:1 (e.g., in the range of 5:1 to 50:1); preferably, the zinc:aluminum molar concentration ratio is in the range of 1:1 to 2:1, 2:1 to 3:1, 3:1 to 4:1, 4:1 to 5:1, 5:1 to 6:1, 6:1 to 8:1, 8:1 to 10:1, 10:1 to 12:1, 12:1 to 15:1, 15:1 to 20:1, 20:1 to 30:1, 30:1 to 40:1, or 40:1 to 50:1;
preferably, in step (2), the soluble salt solution is mixed with the alkaline risedronate solution at a zinc:risedronate molar concentration ratio in the range of 1:1 to 16:1 (e.g., in the range of 2:1 to 16:1); preferably, the zinc:risedronate molar concentration ratio is in the range of 1:1 to 2:1, 2:1 to 4:1, 4:1 to 6:1, 6:1 to 8:1, 8:1 to 10:1, 10:1 to 12:1, 12:1 to 14:1 or 14:1 to 16:1;
preferably, in step (2), the soluble salt solution is mixed with the alkaline risedronate solution in such a manner that zinc ions, aluminum ions and risedronate are co-precipitated;
preferably, zinc ions, aluminum ions and risedronate are precipitated in the manner of coprecipitation, so as to obtain zinc aluminum risedronate particles;
preferably, in step (2), the alkaline risedronate solution is added dropwise to the soluble salt solution, so that zinc ions, aluminum ions and risedronate are co-precipitated;
preferably, the alkaline risedronate solution is selected from the group consisting of solution of risedronic acid and sodium hydroxide, solution of risedronic acid and phosphate (e.g., solution of risedronic acid and disodium hydrogen phosphate, solution of risedronic acid and sodium dihydrogen phosphate), or any combination thereof;
preferably, the soluble salt solution in step (1) is selected from, for example, the group consisting of sulfate solution, chlorate solution, acetate solution, or any combination thereof, preferably chlorate solution or acetate solution.

3. An immunogenic composition, which comprises an immunogen and the adjuvant according to claim 1;
preferably, the immunogen is selected from the group consisting of a protein, nucleic acid, polysaccharide, or immunogenic part thereof;
preferably, the immunogen is derived from a pathogen such as virus, bacterium, fungus;
preferably, the immunogen is a protein or immunogenic fragment thereof; preferably, the immunogen is a protein or immunogenic fragment thereof derived from a pathogen such as virus, bacterium, fungus; preferably, the virus is selected from the group consisting of respiratory virus (e.g., influenza, parainfluenza, rhinovirus, coronavirus, respiratory syncytial virus), enterovirus (e.g., EV71 virus, rotavirus), varicella-zoster virus (VZV); preferably, the immunogen is a coronavirus protein or immunogenic fragment thereof, such as a coronavirus spike protein or immunogenic fragment thereof;
preferably, the coronavirus is selected from the group consisting of Orthocoronavirinae α virus (e.g., 229E and NL63), Orthocoronavirinae β virus (e.g., OC43 and HKU1), severe acute respiratory syndrome-associated coronavirus (SARS-CoV), Middle East respiratory syndrome-associated coronavirus (MERS-CoV), and SARS-CoV-2; preferably, the coronavirus is selected from the group consisting of severe acute respiratory syndrome-associated coronavirus (SARS-CoV), Middle East respiratory syndrome-related coronavirus (MERS-CoV) and SARS-CoV-2, preferably SARS-CoV-2;
preferably, the immunogen is a SARS-CoV-2 S protein or immunogenic fragment thereof, a varicella-zoster virus gE protein or immunogenic fragment thereof, an influenza virus HA protein or immunogenic fragment thereof, or, a rotavirus VP4 protein or immunogenic fragment thereof;
preferably, the immunogenic composition further comprises a pharmaceutically acceptable auxiliary material, such as excipient, preservative, antibacterial agent, buffer and/or additional immune adjuvant; preferably, the additional immune adjuvant is selected from the group consisting of aluminum adjuvant (e.g., aluminum hydroxide), Freund's adjuvant (e.g., complete Freund's adjuvant and incomplete Freund's adjuvant), *Corynebacterium pumilus,* lipopolysaccharide, cytokine, or any combination thereof;
preferably, the immunogenic composition further comprises a second immunogen; preferably, the second immunogen includes but is not limited to a protein, nucleic acid, polysaccharide, or an immunogenic part thereof;
preferably, the immunogenic composition is a vaccine.

4. Use of the adjuvant according to claim 1 for preparing an immunogenic composition, or as a carrier for delivering an immunogen, or as an immunoenhancer for an immunogen, or for enhancing an immunogenicity of an immunogen, or for enhancing an immune response to an immunogen in a subject, or for preparing a preparation for enhancing an immune response to an immunogen in a subject;
preferably, the immunogen is as defined in claim 3;
preferably, the immune response is a cellular immune response and/or a humoral immune response; preferably, the cellular immune response is a T cell immune response; preferably, the T cell immune response is Th1 immune response and/or Th2 immune response.

5. A method for preparing an immunogenic composition, which comprises: a step of mixing the adjuvant according to claim 1 with an immunogen;
preferably, the immunogen is as defined in claim 3;
preferably, the method further comprises a step of adding a pharmaceutically acceptable auxiliary material;
preferably, the auxiliary material is, for example, excipient, preservative, antibacterial agent, buffer and/or additional immune adjuvant; preferably, the additional immune adjuvant is selected from the group consisting of aluminum adjuvant (e.g., aluminum hydroxide), Freund's adjuvant (e.g., complete Freund's adjuvant and incomplete Freund's adjuvant), *Corynebacterium pumilus,* lipopolysaccharide, cytokine, or any combination thereof;
preferably, the method further comprises a step of adding a second immunogen; preferably, the second immunogen includes but not is limited to a protein, nucleic acid, polysaccharide, or an immunogenic part thereof;
preferably, the immunogenic composition is a vaccine.

6. Use of the immunogenic composition according to claim 3 in the manufacture of a medicament for the prevention and/or treatment of a disease in a subject, wherein the disease is a disease that can be prevented or treated by an immune response induced by the immunogen;
preferably, the immunogen is derived from a coronavirus (e.g., SARS-CoV-2), and the disease is a coronavirus (e.g., SARS-CoV-2) infection or a coronavirus (e.g., SARS-CoV-2) infection-associated disease;
preferably, the immunogen is a protein (e.g., spike protein) of a coronavirus (e.g., SARS-CoV-2) or an immunogenic fragment thereof, and the disease is a coronavirus (e.g., SARS-CoV-2) infection or a coronavirus (e.g., SARS-CoV-2) infection-associated disease;
preferably, the immunogen is a structural protein (e.g., S protein) of SARS-CoV-2 or an immunogenic fragment thereof; and, the disease is a SARS-CoV-2 infection or a SARS-CoV-2 infection-associated disease (e.g., pneumonia caused by SARS-CoV-2 (COVID-19));
preferably, the immunogen is derived from a varicella-zoster virus, and the disease is a varicella-zoster virus infection or a varicella-zoster virus infection-associated disease;
preferably, the immunogen is a protein (e.g., gE protein) of varicella-zoster virus or an immunogenic fragment thereof, and the disease is a varicella-zoster virus infection or a varicella-zoster virus infection-associated disease;
preferably, the immunogen is derived from an influenza virus, and the disease is an influenza virus infection or an influenza virus infection-associated disease;
preferably, the immunogen is an influenza virus protein (e.g., HA protein) or an immunogenic fragment thereof, and the disease is an influenza virus infection or an influenza virus infection-associated disease;
preferably, the immunogen is derived from a rotavirus, and the disease is a rotavirus infection or a rotavirus infection-associated disease;
preferably, the immunogen is a rotavirus protein (e.g., VP4 protein) or an immunogenic fragment thereof, and the disease is a rotavirus infection or a rotavirus infection-associated disease;
preferably, the subject is an animal, such as an avian or a mammal;
preferably, the subject is a rodent, porcine, feline, canine, equine, primate or avian;
preferably, the subject is a mouse, mink, guinea pig, Syrian golden hamster or cynomolgus monkey;
preferably, the subject is a human.

7. A method for improving an immunogenicity of an immunogen, which comprises a step of mixing the immunogen with the adjuvant according to claim 1; preferably, the immunogen is as defined in claim 3.

8. A method for stimulating or enhancing an immune response to an immunogen in a subject, which comprises a step of administering to the subject an effective amount of an immunogenic composition containing the immunogen and the adjuvant according to claim 1;
preferably, the immunogen is as defined in claim 3;
preferably, the subject is an animal, such as an avian or a mammal; preferably, the subject is a rodent, porcine, feline, canine, equine, primate or avian; preferably, the subject is a mouse, mink, guinea pig, Syrian golden hamster or cynomolgus monkey; preferably, the subject is a human;
preferably, the immune response is a cellular immune response and/or a humoral immune response; preferably, the cellular immune response is a T cell immune response; preferably, the T cell immune response is Th1 immune response and/or Th2 immune response;
preferably, the immunogenic composition is administered by a route selected from: intramuscular injection, subcutaneous injection, intradermal administration, intranasal administration, oral administration, transdermal or intravenous injection;
preferably, the immunogenic composition is administered by intramuscular injection.

9. A method for preventing or treating a disease, which comprises a step of administering to a subject an effective amount of the immunogenic composition according to claim 3, wherein the disease is a disease that is capable of being prevented or treated by an immune response induced by the immunogen;
preferably, the disease is a coronavirus (e.g., SARS-CoV-2) infection or a coronavirus (e.g., SARS-CoV-2) infection-associated disease, and the immunogen is derived from a coronavirus (e.g., SARS-CoV-2); for example, the immunogen is a protein (e.g., spike protein) of a coronavirus (e.g., SARS-CoV-2) or immunogenic fragment thereof;
preferably, the disease is a SARS-CoV-2 infection or a SARS-CoV-2 infection-associated disease, such as a pneumonia caused by SARS-CoV-2 (COVID-19), and the immunogen is derived from a SARS-CoV-2; for example, the immunogen is a SARS-CoV-2 structural protein (e.g., S protein) or immunogenic fragment thereof;
preferably, the disease is a varicella-zoster virus infection or a varicella-zoster virus infection-associated disease, and the immunogen is derived from a varicella-zoster virus; for example, the immunogen is a varicella-zoster virus protein (e.g., gE protein) or immunogenic fragments thereof;
preferably, the disease is an influenza virus infection or an influenza virus infection-associated disease, and the immunogen is derived from an influenza virus; for example, the immunogen is an influenza virus protein (e.g., HA protein) or immunogenic fragment thereof;
preferably, the disease is a rotavirus infection or a rotavirus infection-associated disease, and the immunogen is derived from a rotavirus; for example, the immunogen is a rotavirus protein (e.g., VP4 protein) or immunogenic fragment thereof;
preferably, the subject is an animal, such as avian or mammal; preferably, the subject is a rodent, porcine, feline, canine, equine, primate or avian; preferably, the subject is a mouse, mink, guinea pig, Syrian golden hamster or cynomolgus monkey; preferably, the subject is a human.
